# EUROPEAN PATENT SPECIFICATION

(11) **EP 2 999 429 B1**
(45) Date of publication and mention of the grant of the patent: **04.07.2018**
(21) Application number: 14801036.6
(22) Date of filing: 18.05.2014
(51) Int. Cl.: A61F 2/06, A61F 2/66, A61F 2/90, A61F 2/07

(54) **ASCENDING AORTA STENT-GRAFT SYSTEM**
AUFSTEIGENDES AORTA-STENTIMPLANTATSYSTEM
SYSTÈME DE GREFFON D'ENDOPROTHÈSE D'AORTE ASCENDANTE

(30) Priority: 23.05.2013 US 201361826544 P
(43) Date of publication of application: 30.03.2016
(73) Proprietor: Endospan Ltd., 46733 Herzilyia Pituach (IL)
(72) Inventor: SHALEV, Alon, Ra'anana 43587 (IL)
(74) Representative: Evens, Paul Jonathan
(86) International application number: PCT/IL2014/050434
(87) International publication number: WO 2014/188412

(56) References cited:
- EP-B1- 1 772 120
- WO-A1-01/52776
- WO-A1-2006/028925
- WO-A1-2007/115017
- WO-A1-2009/104000
- US-A1- 2003 139 805
- US-A1- 2005 273 155
- US-A1- 2009 264 993
- US-A1- 2010 249 899
- US-A1- 2011 218 619
- US-A1- 2013 116 775
- US-B2- 7 628 803

## Description

### CROSS-REFERENCE TO RELATED APPLICATIONS

The present application claims the benefit of US Provisional Application 61/826,544, filed May 23, 2013, which is assigned to the assignee of the present application. The present application relates generally to prostheses, and specifically to tubular prostheses, including endovascular grafts and stent-grafts, and examples of surgical techniques for using the prostheses to maintain patency of body passages such as blood vessels, and treating aneurysms and arterial wall dissections.

### BACKGROUND OF THE APPLICATION

Endovascular prostheses are sometimes used to treat aortic aneurysms. Such treatment includes implanting a stent or stent-graft within the diseased vessel to bypass the anomaly. An aneurysm is a sac formed by the dilation of the wall of the artery. Aneurysms may be congenital, but are usually caused by disease or, occasionally, by trauma. Aortic aneurysms which commonly form between the renal arteries and the iliac arteries are referred to as abdominal aortic aneurysms ("AAAs"). Other aneurysms occur in the aorta, such as thoracic aortic aneurysms ("TAAs") and aortic uni-iliac ("AUI") aneurysms. A TAA may occur downstream the aortic arch, i.e., in the descending aorta. Alternatively, a TAA may occur in the aortic arch itself, where the aorta branches to supply the brachiocephalic, left carotid and subclavian arteries, or may occur in the ascending aorta.

Endo-Vascular Aneurysm Repair (EVAR) has transformed the practice of treatment of aortic aneurysms from an open surgical approach to a much less invasive surgical approach. The first step of an endovascular intervention usually requires introducing a delivery system into the vasculature of a subject. If the crossing profile, i.e., the external diameter, of the delivery system is 14 Fr or lower (3 Fr = 1 millimeter), a true percutaneous approach may be used, because vascular closure devices are available for proper closure of such puncture sites. If the crossing profile at least 15-16 Fr, a vascular cut-down is usually required in advance as a preparatory step to introduction of the delivery system.

Blood vessels occasionally weaken or even rupture. For example, in the aortic artery, the vascular wall can weaken or tear, resulting in dangerous conditions such as aneurysm and dissection. Treatment of such conditions can be performed by implanting a prosthesis within the vascular system using minimally-invasive surgical procedures. An endoluminal prosthesis typically includes one or more stents affixed to graft material and is delivered to the treatment site by endovascular insertion. Once the endoluminal prosthesis is radially enlarged, it should remain in place indefinitely by self-attachment to the vessel wall, acting as a substitute vessel for the flow of blood or other fluids.

Aortic dissection is a tear or partial tear in the inner wall of the aorta, which causes blood to flow between the layers of the wall of the aorta, forcing the layers apart. Aortic dissections may be divided into two types in accordance with the Stanford classification: Type A dissections involve the ascending aorta and/or aortic arch, and possibly the descending aorta. Type B dissections involve the descending aorta or the arch (distal to right brachiocephalic artery origin), without involvement of the ascending aorta.

Document WO 2009/104000 discloses an apparatus comprising a stent-graft and a delivery tool, the stent graft comprising a support element and a covering element comprising proximally-extending pieces defining fenestrations.

### SUMMARY OF THE APPLICATION

Insofar as the terms "invention" and/or "embodiment" are used in the following, and/ or features are presented as being optional, this should be interpreted in such a way that the only protection sought is that of the invention as claimed.

In some applications of the present invention, a multi-component stent-graft system is provided for treating an ascending aorta suffering from an aneurysm or a dissection. The system is configured to be deployed in the ascending aorta, aortic sinuses, and left and right coronary arteries. Upon deployment, the multi-component stent-graft system defines blood-flow paths both through the ascending aorta and into the coronary arteries. The multi-component stent-graft system comprises a generally tubular main stent-graft, and, typically, two generally tubular branching covered stents. When the stent-graft is unconstrained in a radially-expanded state, a proximal end portion of a covering element thereof is shaped so as to define at least first and second proximally-extending pieces. The proximally-extending pieces are configured to be positioned at least partially in the aortic sinuses so as to provide a proximal landing zone for the stent-graft.

When the stent-graft is unconstrained in its radially-expanded state and the proximally-extending pieces are fully proximally extended, the proximally-extending pieces typically:
- are shaped so as to define respective distal bases, which (a) have respective base lengths measured circumferentially around stent-graft, and (b) circumferentially circumscribe respective base arcs, each of which has an angle of between 100 and 140 degrees;
- are shaped so as to define respective proximal-most portions, which are more proximal than all other respective portions of the covering element that circumscribe the respective base arcs; and
- have respective axial lengths, measured axially between the respective distal bases and respective the proximal-most portions, which lengths equal between 50% and 150% of the respective base lengths.

For some applications, the first and the second proximally-extending pieces are shaped so as to define respective fenestrations through the covering element. The first and the second branching covered stents have respective end portions that are sized and configured to form blood-impervious seals with the respective fenestrations. For some applications, when the stent-graft is unconstrained in the radially-expanded state, the proximal end portion of the covering element is shaped so as to define a third proximally-extending piece, which typically does not define a fenestration.

In some applications of the present invention, a delivery tool is provided to convey the stent-graft in the radially-compressed state to a target location in vasculature of a subject, and deploy the stent-graft at the target location. The delivery tool comprises at least one inner shaft and an external sheath. The at least one inner shaft is shaped so as to define a primary bore and first and second secondary bores therethrough. For delivery, the stent-graft is removably positioned such that the distal stent-graft end surrounds an axial portion of the at least one inner shaft.

For some applications, the at least one inner shaft comprises (a) a primary inner shaft, which is shaped so as to define the primary bore therethrough, and (b) first and second secondary inner shafts, which are shaped so as to define the first and the second secondary bores therethrough, respectively. Typically, for delivery the stent-graft is removably positioned such that (a) the distal stent-graft end surrounds respective axial portions of the primary inner shaft and the first and the second secondary inner shafts, (b) the proximal stent-graft end surrounds an axial portion of the primary inner shaft, and (c) the first and the second secondary inner shafts extend proximally beyond the proximal stent-graft end.

For some applications in which the first and the second proximally-extending pieces are shaped so as to define the respective fenestrations, the first and the second secondary inner shafts pass through the first and the second fenestrations, respectively, when the stent-graft is removably positioned such that the first and the second secondary inner shafts extend proximally beyond the proximal stent-graft end. This positioning of the secondary inner shafts enables the threading of secondary guidewires through the fenestrations, as described hereinbelow.

Typically, the delivery tool comprises a proximal tip that is coupled to a proximal end portion of the at least one inner shaft, typically of the primary inner shaft when provided. The proximal tip is configured to reduce potential damage to a blood vessel wall when the delivery tool is proximally translated relative to the vessel wall.

For some applications, an external surface of the proximal tip is shaped so as to define first and second grooves. The grooves extend axially along at least an axial portion of the tip, and are shaped and sized so as to reversibly receive the proximal end portions of the first and the second secondary inner shafts, respectively. Typically, the grooves reach a distal end of the proximal tip. For some applications, the grooves taper from their distal ends toward their proximal ends. Typically, the proximal tip is shaped so as to define a tip bore therethrough, and the tip bore and the primary bore are arranged axially continuously.

During an implantation procedure using the delivery tool, a primary guidewire and two secondary guidewires are endovascularly (typically percutaneously) introduced into the vasculature. The guidewires are advanced to the ascending aorta, typically via the descending aorta. The primary guidewire is typically advanced between leaflets of an aortic valve into a left ventricle. The secondary guidewires are advanced into left and right coronary arteries, respectively.

Outside the patient's body, a distal end of the primary guidewire is threaded through the tip bore and the primary bore, and respective distal ends of the secondary guidewires are threaded through the first and the second secondary bores, respectively. The delivery tool is advanced over the three guidewires into the ascending aorta, while the stent-graft is removably positioned, while in the radially-compressed state, within the external sheath.

The external sheath is distally axially translated, so as to facilitate a partial transition of the stent-graft from the radially-compressed state to the radially-expanded state. The secondary guidewires guide the respective fenestrations of the proximally-extending pieces to the respective left and right coronary ostia, and align the fenestrations with the ostia. Such alignment facilitates the deployment of the branching covered stents in the coronary arteries, as described below. The external sheath is further distally axially translated, so as to facilitate the remainder of the transition of the stent-graft from the radially-compressed state to the radially-expanded state. As a result of the deployment, the proximally-extending pieces, as well as the third proximally-extending piece, if provided, are positioned at least partially in the aortic sinuses, respectively, so as to provide a proximal landing zone for the stent-graft. A relatively long landing zone of blood vessel wall, such as about 3 cm, is desirable to provide good anchoring and sealing in the ascending aorta, because the ascending aorta is highly motile and pulsatile. The proximal-most portions of the proximally-extending pieces, and of the third proximally-extending piece if provided, are positioned in the aortic sinuses, respectively. The fenestrations are aligned with the coronary ostia. The delivery tool is removed from the vasculature.

The branching covered stents are separately deployed in the left and the right coronary arteries, respectively, and are coupled to the stent-graft so as to form blood-impervious seals with the respective fenestrations. Upon full deployment, the stent-graft and the branching covered stents together provide blood-flow paths (a) through the ascending aorta, bypassing the aneurysm or dissection, and (b) to the left and the right coronary arteries.

There is therefore provided, in accordance with an application of the present invention, apparatus including a generally tubular stent-graft, which has distal and proximal stent-graft ends and includes:
a generally tubular support element, which includes a plurality of structural stent elements; and
a covering element that is attached to and at least partially covers the support element,
wherein when the stent-graft is unconstrained in a radially-expanded state, a proximal end portion of the covering element is shaped so as to define at least first and second proximally-extending pieces, and
wherein when the stent-graft is unconstrained in the radially-expanded state and the proximally-extending pieces are fully proximally extended, the proximally-extending pieces:
   are shaped so as to define respective distal bases, which (a) have respective base lengths measured circumferentially around the stent-graft, and (b) circumferentially circumscribe respective base arcs, each of which base arcs has an angle of between 100 and 140 degrees,
   are shaped so as to define respective proximal-most portions, which are more proximal than all other respective portions of the covering element that circumscribe the respective base arcs, and
   have respective axial lengths, measured axially between the respective distal bases and the respective proximal-most portions, which lengths equal between 50% and 150% of the respective base lengths.

For some applications, when fully proximally extended, the first and the second proximally-extending pieces have respective surface areas equal to at least 15% of the square of the respective base lengths.

For some applications, the first and the second proximally-extending pieces have substantially a same shape and size.

For some applications, one or more proximal-most points of the covering element coincide with the proximal stent-graft end.

For some applications, the first proximally-extending piece is attached to one or more of the structural stent elements, and the second proximally-extending piece is attached to one or more of the structural stent elements.

For some applications, the respective axial lengths of the proximally-extending piece are between 15 mm and 30 mm, when the stent-graft is unconstrained in the radially-expanded state and the proximally-extending pieces are fully proximally extended.

For some applications, the proximally-extending pieces are outwardly convex when the stent-graft is unconstrained in the radially-expanded state.

For some applications, the proximally-extending pieces are outwardly conically flared at an angle of between 10 and 30 degrees with a central longitudinal axis of the stent-graft, when the stent-graft is unconstrained in the radially-expanded state and the proximally-extending pieces are fully proximally extended.

For any of the applications described above, the first and the second proximally-extending pieces may be shaped so as to define respective fenestrations through the covering element, each of which fenestrations has an area of at least 100 mm2 when the stent-graft is unconstrained in the radially-expanded state and the proximally-extending pieces are fully proximally extended.

For some applications, the apparatus includes a multi-component stent-graft system, which includes the stent-graft and first and second branching covered stents, which have respective end portions that are sized and configured to form blood-impervious seals with the fenestrations of the first and the second proximally-extending pieces of the stent-graft, respectively.

For some applications, one or more of the structural stent elements are attached to each of the first and the second proximally-extending pieces, such that at least a portion of the one or more of the structural stent elements is proximal to the fenestrations when the stent-graft is unconstrained in the radially-expanded state and the proximally-extending pieces are fully proximally extended.

For some applications, each of the fenestrations is generally circular when the stent-graft is unconstrained in the radially-expanded state and the proximally-extending pieces are fully proximally extended. For some applications, a diameter of each of the fenestrations is between 3 and 8 mm, when the stent-graft is unconstrained in the radially-expanded state and the proximally-extending pieces are fully proximally extended.

For some applications, the fenestrations are circumferentially centered on the respective proximally-extending pieces when the stent-graft is unconstrained in the radially-expanded state and the proximally-extending pieces are fully proximally extended.

For some applications, respective closest distances of the fenestrations to the proximal-most portions equal between 10% and 30% of the respective axial lengths, when the stent-graft is unconstrained in the radially-expanded state and the proximally-extending pieces are fully proximally extended.

For some applications, the stent-graft further includes a radiopaque wire that is securely mounted around one of the fenestrations.

For any of the applications described above, the first and the second proximally-extending pieces may be shaped so as to define respective scallops, which have respective axial lengths measured from respective distal-most portions of the scallops to the respective proximal most-portions of the respective proximally-extending pieces, each of which axial lengths is at least 7 mm when the stent-graft is unconstrained in the radially-expanded state and the proximally-extending pieces are fully proximally extended. For some applications, one or more of the structural stent elements are attached to each of the first and the second proximally-extending pieces such that respective portions of at least one of the one or more of the structural stent elements traverse the scallops when the stent-graft is unconstrained in the radially-expanded state and the proximally-extending pieces are fully proximally extended.

For any of the applications described above, the first and the second proximally-extending pieces may be shaped as first and second lobes, respectively, when the stent-graft is unconstrained in the radially-expanded state and the proximally-extending pieces are fully proximally extended. For some applications, the first and the second lobes are semicircular, when the stent-graft is unconstrained in the radially-expanded state and the lobes are fully proximally extended. For some applications, each of the first and the second semicircular lobes are shaped so as to circumscribe approximately 180 degrees of a circle, when the stent-graft is unconstrained in the radially-expanded state and the lobes are fully proximally extended. For some applications, each of the first and the second semicircular lobes are shaped so as to circumscribe 100 to 170 degrees of a circle, when the stent-graft is unconstrained in the radially-expanded state and the lobes are fully proximally extended. For some applications, each of the first and the second semicircular lobes are shaped so as to circumscribe approximately 190 to 270 of a circle, when the stent-graft is unconstrained in the radially-expanded state and the lobes are fully proximally extended.

For any of the applications described above, an arc between circumferentially nearest portions of the first and the second proximally-extending pieces may have an angle of no more than 50 degrees, when the stent-graft is unconstrained in the radially-expanded state. For some applications, the angle of the arc between the circumferentially nearest portions is at least 5 degrees, when the stent-graft is unconstrained in the radially-expanded state.

For any of the applications described above,
when the stent-graft is unconstrained in the radially-expanded state, the proximal end portion of the covering element may be shaped so as to define a third proximally-extending piece, and
when the stent-graft is unconstrained in the radially-expanded state and the third proximally-extending piece is fully proximally extended, the third proximally-extending piece:
may be shaped so as to define a third distal base, which (a) has a third base length measured circumferentially around the stent-graft, and (b) circumferentially circumscribes a third base arc, which third base arc has an angle of between 100 and 140 degrees,
may be shaped so as to define a third proximal-most portion, which is more proximal than all other portions of the covering element that circumscribe the third base arc, and
may have a third axial length, measured axially between the third distal base and the third proximal-most portion, which length equals between 50% and 150% of the third base length.

For some applications, the first and the second proximally-extending pieces are shaped so as to define respective fenestrations through the covering element, each of which fenestrations has an area of at least 6 mm2 when the stent-graft is unconstrained in the radially-expanded state and the proximally-extending pieces are fully proximally extended; and the third proximally-extending piece is not shaped so as to define any fenestrations through the covering element.

For some applications, the first and the second proximally-extending pieces are shaped so as to define respective scallops, which have respective axial lengths measured from respective distal-most portions to the respective proximal most-portions of the respective proximally-extending pieces, each of which axial lengths is at least 7 mm when the stent-graft is unconstrained in the radially-expanded state and the proximally-extending pieces are fully proximally extended; and the third proximally-extending piece is not shaped so as to define any scallops.

For any of the applications described above, the apparatus may further include a delivery tool, which (a) is configured to convey the stent-graft in the radially-compressed state to a target location in vasculature of a subject, and deploy the stent-graft at the target location, and (b) includes (i) at least one inner shaft, which is shaped so as to define a primary bore and first and second secondary bores therethrough; and (ii) an external sheath,
the stent-graft may be removably positioned, while in the radially-compressed state, within the external sheath, such that the distal stent-graft end surrounds an axial portion of the at least one inner shaft, and
the delivery tool may be is configured such that axial translation of the external sheath facilitates a transition of the stent-graft from the radially-compressed state to the radially-expanded state.

For some applications, the at least one inner shaft includes: a primary inner shaft, which is shaped so as to define the primary bore therethrough; and first and second secondary inner shafts, which are shaped so as to define the first and the second secondary bores therethrough, respectively, and the stent-graft is removably positioned, while in the radially-compressed state, within the external sheath, such that (a) the distal stent-graft end surrounds respective axial portions of the primary inner shaft and the first and the second secondary inner shafts, (b) the proximal stent-graft end surrounds an axial portion of the primary inner shaft, and (c) the first and the second secondary inner shafts extend proximally beyond the proximal stent-graft end.

For some applications, the first and the second proximally-extending pieces are shaped so as to define respective first and second fenestrations through the covering element, and the first and the second secondary inner shafts pass through the first and the second fenestrations, respectively, when the stent-graft is removably positioned such that the first and the second secondary inner shafts extend proximally beyond the proximal stent-graft end.

For some applications, the first and the second proximally-extending pieces are shaped so as to define respective first and second scallops, and the first and the second secondary inner shafts pass through the first and the second scallops, respectively, when the stent-graft is removably positioned such that the first and the second secondary inner shafts extend proximally beyond the proximal stent-graft end.

For some applications, the delivery tool further includes a proximal tip coupled to a proximal end portion of the primary inner shaft, and an external surface of the proximal tip is shaped so as to define first and second grooves, which (a) extend axially along at least an axial portion of the tip, and (b) are shaped and sized so as to reversibly receive respective proximal end portions of the first and the second secondary inner shafts.

For some applications, the delivery tool further includes at least one stent-graft support member which is securely fixed to an external surface of the primary inner shaft, and which is configured to prevent distal axial translation of the stent-graft as the external sheath is distally axially translated to facilitate the transition of the stent-graft from the radially-compressed state to the radially-expanded state.

For some applications, the stent-graft support member is circumferentially disposed around the primary inner shaft.

For some applications, the stent-graft support member is positioned proximally adjacent to the proximal stent-graft end, when the stent-graft is removably positioned, while in the radially-compressed state, within the external sheath with the proximal stent-graft end surrounding the axial portion of the primary inner shaft.

For some applications, the delivery tool further includes a proximal tip coupled to a proximal end portion of the at least one inner shaft. For some applications, the proximal tip is conically shaped, such that the tip has a smallest cross-sectional area at a proximal-most portion of the tip. Alternatively, for some applications, the proximal tip is hemispherically shaped, such that the tip has a smallest cross-sectional area at a proximal-most portion of the tip. For some applications, the proximal tip is shaped so as to define a tip bore therethrough, and the tip bore and the primary bore are arranged axially continuously.

For any of the applications described above, a diameter of the stent-graft may be between 30 and 48 mm, such as between 35 and 45 mm, when the stent-graft is unconstrained in the radially-expanded state.

For any of the applications described above, the structural stent elements may include a metal, such as an elastic metal, e.g., a superelastic alloy, e.g., Nitinol. For some applications, the elastic metal includes stainless steel.

For any of the applications described above, the covering element may include polyester, such as polyethylene terephthalate (PET) and/or expanded polytetrafluoroethylene (ePTFE).

For any of the applications described above, the stent-graft may further include one or more radiopaque markers, which are securely mounted to the stent-graft to distinguish between the first and the second proximally-extending pieces. For some applications, at least a first one of the radiopaque markers is positioned on the first proximally-extending piece. For some applications, at least a second one of the radiopaque markers is positioned on the second proximally-extending piece. For some applications, the first and the second radiopaque markers have different respective shapes.

There is further provided, in accordance with an application of the present invention, apparatus for delivering at least a first stent-graft, the apparatus including a delivery tool, which (a) is configured to convey the first stent-graft in a radially-compressed state to a target location in vasculature of a subject, and (b) includes:
a primary inner shaft, which is shaped so as to define a primary bore therethrough;
a proximal tip, which is coupled to a proximal end portion of the primary inner shaft, and which has an external surface that is shaped so as to define at least one groove, which extends axially along at least an axial portion of the tip; and
at least one inner shaft, which (a) is shaped so as to define a secondary bore therethrough, and (b) has a proximal end portion that is removably positioned at least partially within the at least one groove.

For some applications:
the at least one groove includes first and second grooves,
the at least one inner shaft includes first and second inner shafts,
the secondary bore includes first and second secondary bores,
the external surface of the proximal tip is shaped so as to define the first and the second grooves, which extend axially along the at least an axial portion of the tip, and
the first and the second inner shafts (a) are shaped so as to define the first and the second secondary bores therethrough, respectively, and (b) have respective first and second end portions that are removably positioned at least partially within the first and the second grooves, respectively.

For some applications, the delivery tool further includes an external sheath, which is removably disposed surrounding (a) the primary inner shaft and (b) a distal portion of the at least one secondary inner shaft, such that a proximal end of the at least one secondary inner shaft extends proximally from the external sheath. For some applications, the external sheath is configured to hold the proximal end portion of the at least one secondary inner shaft in place when the external sheath is removably disposed surrounding (a) the primary inner shaft and (b) the distal portion of the at least one second secondary inner shaft. For some applications, the proximal end portion of the at least one secondary inner shaft extends radially no more than does an external surface of the external sheath, when the external sheath is removably disposed surrounding (a) the primary inner shaft and (b) the distal portion of the at least one second secondary inner shaft.

For any of the applications described above, the proximal tip may be conically shaped, such that the tip has a smallest cross-sectional area at a proximal-most portion of the tip. Alternatively, for any of the applications described above, the proximal tip may be hemispherically shaped, such that the tip has a smallest cross-sectional area at a proximal-most portion of the tip.

For any of the applications described above, the proximal tip may be shaped so as to define a tip bore therethrough, and the tip bore and the primary bore may be arranged axially continuously.

For any of the applications described above, the apparatus may further include the at least a first stent-graft.

There is still further provided an example of a method including:
providing a generally tubular stent-graft, which has distal and proximal stent-graft ends and includes: (a) a generally tubular support element, which includes a plurality of structural stent elements; and (b) a covering element that is attached to and at least partially covers the support element, wherein when the stent-graft is unconstrained in a radially-expanded state, a proximal end portion of the covering element is shaped so as to define at least first and second proximally-extending pieces, and wherein when the stent-graft is unconstrained in the radially-expanded state and the proximally-extending pieces are fully proximally extended, the proximally-extending pieces: (i) are shaped so as to define respective distal bases, which (a) have respective base lengths measured circumferentially around the stent-graft, and (b) circumferentially circumscribe respective base arcs, each of which base arcs has an angle of between 100 and 140 degrees, (ii) are shaped so as to define respective proximal-most portions, which are more proximal than all other respective portions of the covering element that circumscribe the respective base arcs, and (iii) have respective axial lengths, measured axially between the respective distal bases and the respective proximal-most portions, which lengths equal between 50% and 150% of the respective base lengths; and
implanting the stent-graft in an ascending aorta of a subject, such that the proximal-most portions of the proximally-extending pieces are positioned in respective aortic sinuses of the subject.

For some applications, providing the stent-graft includes providing the stent-graft in which the first and the second proximally-extending pieces are shaped so as to define respective fenestrations through the covering element, each of which fenestrations has an area of at least 100 mm2 when the stent-graft is unconstrained in the radially-expanded state and the proximally-extending pieces are fully proximally extended; and implanting the stent-graft includes implanting the stent-graft such that the fenestrations are aligned with left and right coronary ostia of the subject, respectively. In some examples, the method further includes providing first and second branching covered stents; and implanting the first and the second branching covered stents in left and right coronary arteries of the subject, respectively, and coupling respective end portions thereof to form blood-impervious seals with the fenestrations of the first and the second proximally-extending pieces of the stent-graft, respectively.

For some applications, providing the stent-graft includes providing the stent-graft in which the first and the second proximally-extending pieces are shaped so as to define respective scallops, which have respective axial lengths measured from respective distal-most portions of the scallops to the respective proximal most-portions of the respective proximally-extending pieces, each of which axial lengths is at least 7 mm when the stent-graft is unconstrained in the radially-expanded state and the proximally-extending pieces are fully proximally extended; and implanting the stent-graft includes implanting the stent-graft such that the scallops are aligned with left and right coronary ostia of the subject, respectively.

For some applications, providing the stent-graft includes providing the stent-graft in which, when the stent-graft is unconstrained in the radially-expanded state, the proximal end portion of the covering element is shaped so as to define a third proximally-extending piece, and when the stent-graft is unconstrained in the radially-expanded state and the third proximally-extending piece is fully proximally extended, the third proximally-extending piece: (i) is shaped so as to define a third distal base, which (a) has a third base length measured circumferentially around the stent-graft, and (b) circumferentially circumscribes a third base arc, which third base arc has an angle of between 100 and 140 degrees, (ii) is shaped so as to define a third proximal-most portion, which is more proximal than all other portions of the covering element that circumscribe the third base arc, and (iii) has a third axial length, measured axially between the third distal base and the third proximal-most portion, which length equals between 50% and 150% of the third base length; and implanting includes implanting the stent-graft in the ascending aorta, such that the third proximal-most portion is positioned in an aortic sinus of the subject other than the aortic sinuses in which the first and the second proximally-extending pieces are positioned.

For some examples, implanting the stent-graft includes using a delivery tool, advancing the stent-graft to the ascending aorta while removably positioned in the radially-compressed state within an external sheath of the delivery tool, such that the distal stent-graft end surrounds an axial portion of at least one inner shaft of the delivery tool, which at least one inner shaft is shaped so as to define a primary bore and first and second secondary bores therethrough; and deploying the stent-graft in the ascending aorta by axially translating the external sheath so as to transition the stent-graft from the radially-compressed state to the radially-expanded state.

For some applications, the at least one inner shaft includes a primary inner shaft, which is shaped so as to define the primary bore therethrough, and first and second secondary inner shafts, which are shaped so as to define the first and the second secondary bores therethrough, respectively; and advancing the stent-graft includes advancing the stent-graft while removably positioned in the radially-compressed state within the external sheath, such that (a) the distal stent-graft end surrounds respective axial portions of the primary inner shaft and the first and the second secondary inner shafts, (b) the proximal stent-graft end surrounds an axial portion of the primary inner shaft, and (c) the first and the second secondary inner shafts extend proximally beyond the proximal stent-graft end.

For some applications, the first and the second proximally-extending pieces are shaped so as to define respective first and second fenestrations through the covering element, and advancing the stent-graft includes advancing the stent-graft while removably positioned in the radially-compressed state within the external sheath, such that the first and the second secondary inner shafts pass through the first and the second fenestrations, respectively.

For some applications, the first and the second proximally-extending pieces are shaped so as to define respective first and second scallops, and advancing the stent-graft includes advancing the stent-graft while removably positioned in the radially-compressed state within the external sheath, such that the first and the second secondary inner shafts pass through the first and the second scallops, respectively.

For some applications, the delivery tool further includes a proximal tip coupled to a proximal end portion of the primary inner shaft; an external surface of the proximal tip is shaped so as to define first and second grooves, which (a) extend axially along at least an axial portion of the tip, and (b) are shaped and sized so as to reversibly receive respective proximal end portions of the first and the second secondary inner shafts; and advancing the stent-graft includes advancing the stent-graft while the proximal end portion of the first and the second secondary inner shafts are reversibly positioned at least partially within the first and the second grooves, respectively.

For some applications, the delivery tool further includes at least one stent-graft support member which is securely fixed to an external surface of the primary inner shaft, and which is configured to prevent distal axial translation of the stent-graft as the external sheath is distally axially translated to facilitate the transition of the stent-graft from the radially-compressed state to the radially-expanded state.

There is additionally provided an example of a method, not part of the invention, including:
using a delivery tool, conveying at least a first stent-graft to a target location in vasculature of a subject while removably positioned in a radially-compressed state in an external sheath of the delivery tool, which external sheath is removably disposed surrounding (a) a primary inner shaft of the delivery tool, which primary inner shaft is shaped so as to define a primary bore therethrough, and (b) a distal portion of at least one secondary inner shaft of the delivery tool, which at least one secondary inner shaft is shaped so as to define a secondary bore therethrough such that a proximal end of the at least one secondary inner shaft extends proximally from the sheath, wherein the delivery tool includes a proximal tip, which is coupled to a proximal end portion of the primary inner shaft, and which has an external surface that is shaped so as to define at least one groove, which extends axially along at least an axial portion of the tip, and wherein a proximal end portion the at least one secondary inner shaft is removably positioned at least partially within the at least one groove; and
deploying the stent-graft at the target location by axially translating the external sheath so as to transition the stent-graft from the radially-compressed state to a radially-expanded state.

For some applications:
the at least one groove includes first and second grooves,
the at least one inner shaft includes first and second inner shafts,
the secondary bore includes first and second secondary bores,
the external surface of the proximal tip is shaped so as to define the first and the second grooves, which extend axially along the at least an axial portion of the tip,
the first and the second inner shafts are shaped so as to define the first and the second secondary bores therethrough, respectively, and
conveying includes conveying the at least a first stent-graft using the delivery tool while respective first and second end portions of the first and the second inner shafts are removably positioned at least partially within the first and the second grooves, respectively.

The present invention will be more fully understood from the following detailed description of embodiments thereof, taken together with the drawings, in which:

### BRIEF DESCRIPTION OF THE DRAWINGS

Figs. 1A-B are schematic illustration of a multi-component stent-graft system, in accordance with respective applications of the present invention;
Figs. 2A-C are schematic illustrations of respective configurations of a stent-graft of the stent-graft system of Figs. 1A-B, in accordance with respective applications of the present invention;
Fig. 3 is a schematic illustration of a single proximally-extending piece of the stent-graft of Figs. 2A-C, in accordance with an application of the present invention;
Figs. 4A-B are schematic illustrations of components of a delivery tool, upon which the stent-graft of Figs. 2A-C is removably positioned, in accordance with respective applications of the present invention;
Figs. 5A and 5B are schematic illustrations of a portion of inner shafts of the delivery tool of Figs. 4A-B, in accordance with respective applications of the present invention;
Figs. 6A, 6B, and 6C are schematic illustrations of a proximal tip of the delivery tool of Figs. 4A-B, in accordance with respective applications of the present invention;
Figs. 7A-D are schematic illustrations of a portion of a deployment using the delivery tool of Figs. 4A-B, in accordance with an application of the present invention;
Figs. 8A and 8B are schematic illustrations of additional configurations of the delivery tool of Figs. 4A-B, in accordance with respective applications of the present invention; and
Figs. 9A-K are schematic illustrations of an exemplary method of deploying the stent-graft and two branching covered stents in an ascending aorta, using the delivery tool of Figs. 4A-B.

### DETAILED DESCRIPTION OF APPLICATIONS

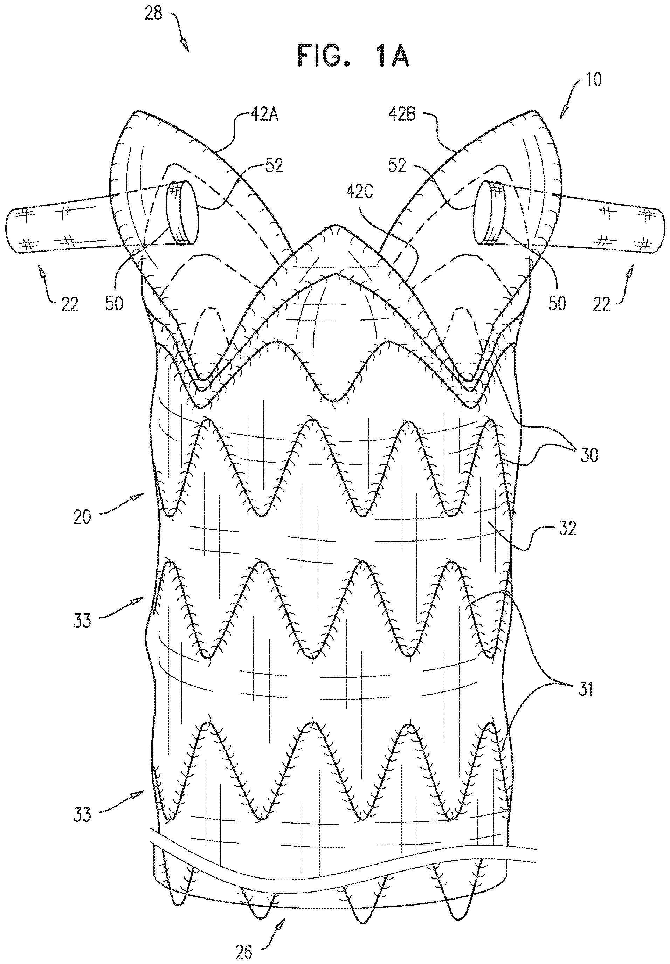

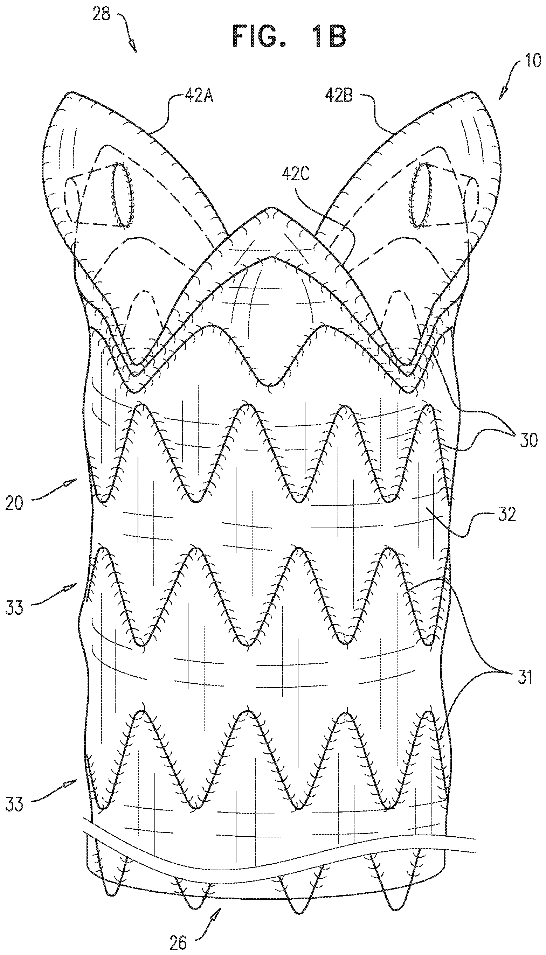

Figs. 1A-B are schematic illustrations of a multi-component stent-graft system 10, in accordance with respective applications of the present invention. In some applications of the present invention, multi-component stent-graft system 10 is provided for treating an ascending aorta suffering from an aneurysm or a dissection (a Type A dissection). The system is configured to be deployed in the ascending aorta, aortic sinuses, and left and right coronary arteries. Upon deployment, the multi-component stent-graft system defines blood-flow paths both through the ascending aorta and into the coronary arteries.

Multi-component stent-graft system 10 comprises a generally tubular main stent-graft 20, and, typically, two generally tubular branching covered stents 22. The main stent-graft and covered stents are configured to assume radially-compressed states, such as when initially positioned in one or more delivery tools, and to assume radially-expanded states upon being deployed in respective target locations in vasculature of a subject. Figs. 1A-B show stent-graft 20 and branching covered stents 22 unconstrained in their radially-expanded states, i.e., no forces are applied to the stent-graft or the branching covered stents by a delivery tool, walls of a blood vessel, or otherwise. For some applications, the stent-graft and branching covered stents are relaxed in their radially-expanded states. For some applications, the stent-graft and branching covered stents are configured to be self-expanding. For example, they may be heat-set to assume their radially-expanded states. Stent-graft 20 has distal and proximal stent-graft ends 26 and 28.

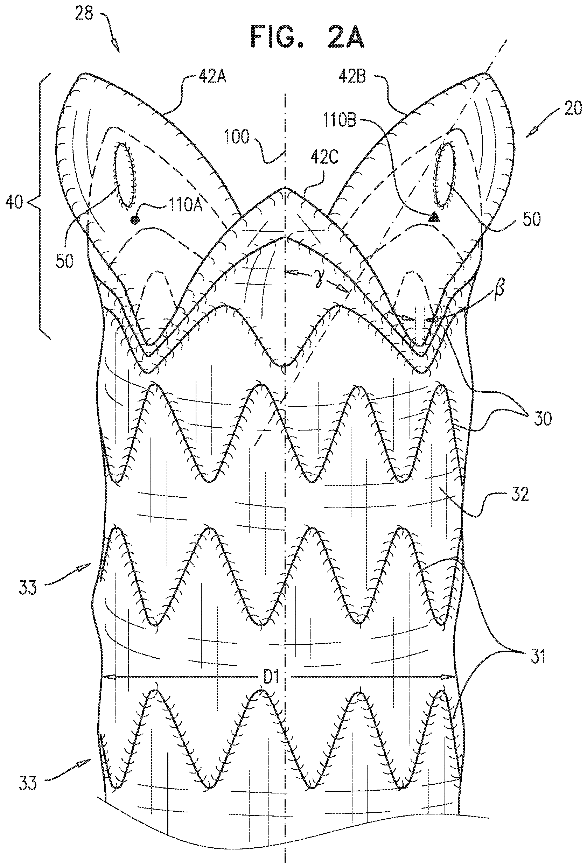

Reference is now made to Figs. 2A-C, which are schematic illustrations of respective configurations of stent-graft 20, in accordance with respective applications of the present invention. Stent-graft 20 comprises a generally tubular support element 30 and a covering element 32 that is attached to and at least partially covers (e.g., only partially covers) the support element. Support element 30 typically comprises a plurality of structural stent elements 31. For some applications, structural stent elements 31 are arranged as a plurality of circumferential stent springs 33. For some applications, support element 30 comprises a metal (such as an elastic metal, or stainless steel), a super-elastic alloy (such as Nitinol). Covering element 32 serves as a blood flow guide through at least a portion of the stent-graft. Covering element 32 typically comprises at least one biologically-compatible substantially blood-impervious flexible sheet, which is attached (such as by stitching) to at least a portion of the respective support element, on either side of the surfaces defined by the support element. The flexible sheet may comprise, for example, a polymeric material (e.g., polyester, or polytetrafluoroethylene), a textile material (e.g., polyethylene terephthalate (PET), or expanded polytetrafluoroethylene (ePTFE)), natural tissue (e.g., saphenous vein or collagen), or a combination thereof. For some applications, such as shown in Figs. 1A-B and 2A-B, one or more proximal-most points of covering element 32 coincide with proximal stent-graft end 28.

For some applications, a diameter D1 (labeled in Fig. 2A) of stent-graft 20 is at least 30 mm, no more than 48 mm, and/or between 30 and 48 mm, such as at least 35 mm, no more than 45 mm, and/or between 35 and 45 mm, when stent-graft 20 is unconstrained in the radially-expanded state.

Each of branching covered stents 22 comprises a generally tubular support element and a covering element that is attached to and at least partially covers (e.g., only partially covers) the support element. The support element typically comprises a plurality of structural stent elements. For some applications, the structural stent elements are arranged as a plurality of circumferential stent springs. For some applications, the support element comprises a metal (such as an elastic metal, or stainless steel), a super-elastic alloy (such as Nitinol). The covering element serves as a blood flow guide through at least a portion of the branching covered stent. The covering element typically comprises at least one biologically-compatible substantially blood-impervious flexible sheet, which is attached (such as by stitching) to at least a portion of the respective support element, on either side of the surfaces defined by the support element. The flexible sheet may comprise, for example, a polymeric material (e.g., polyester, or polytetrafluoroethylene), a textile material (e.g., polyethylene terephthalate (PET), or expanded polytetrafluoroethylene (ePTFE)), natural tissue (e.g., saphenous vein or collagen), or a combination thereof.

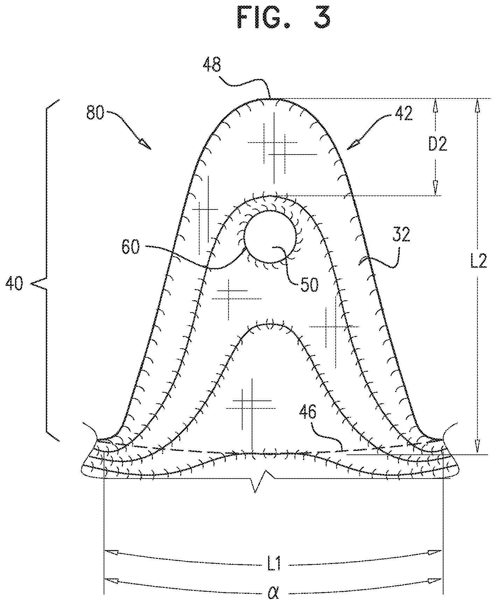

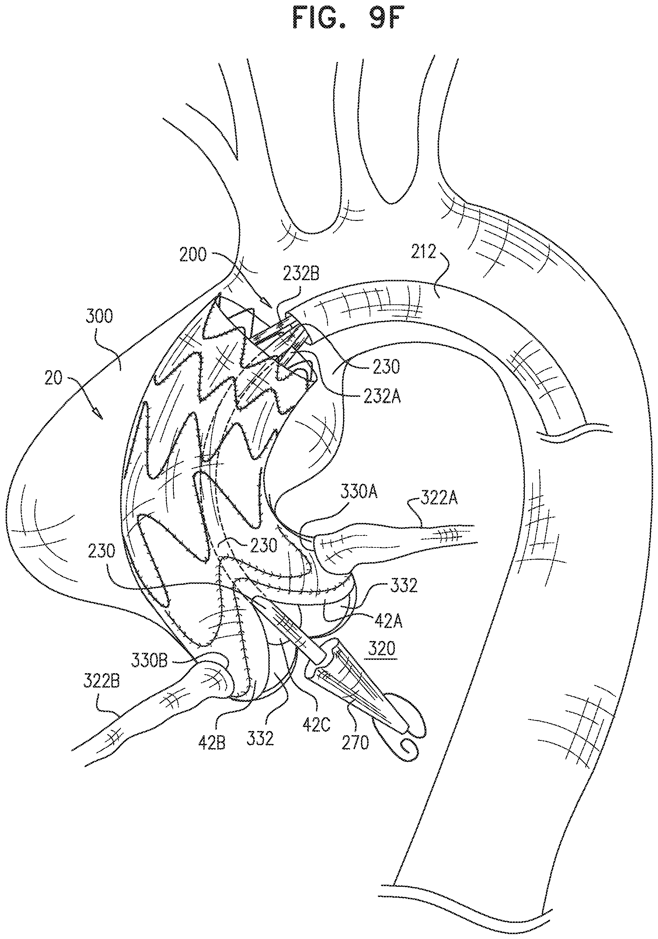

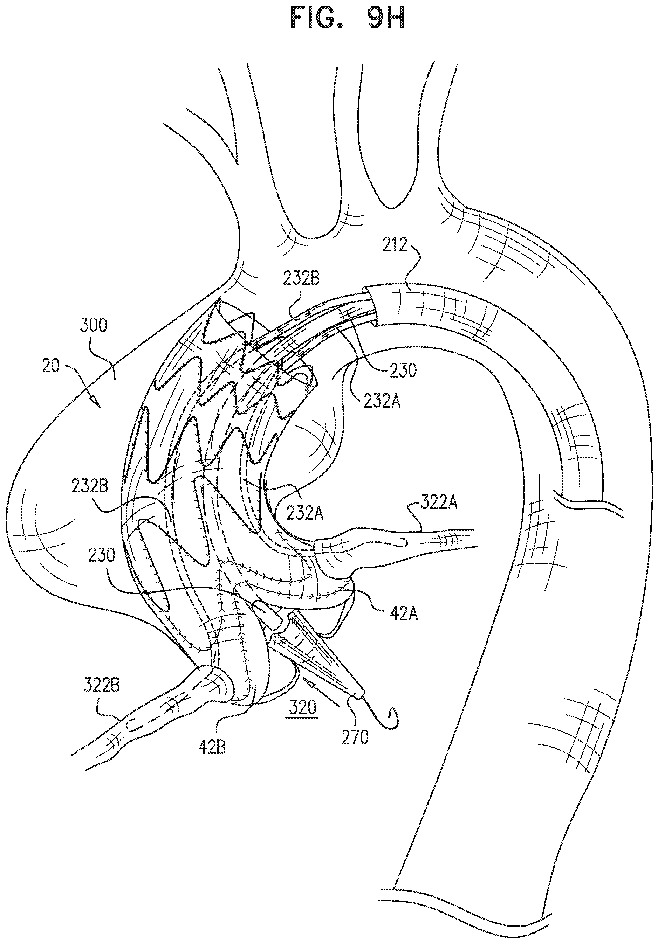

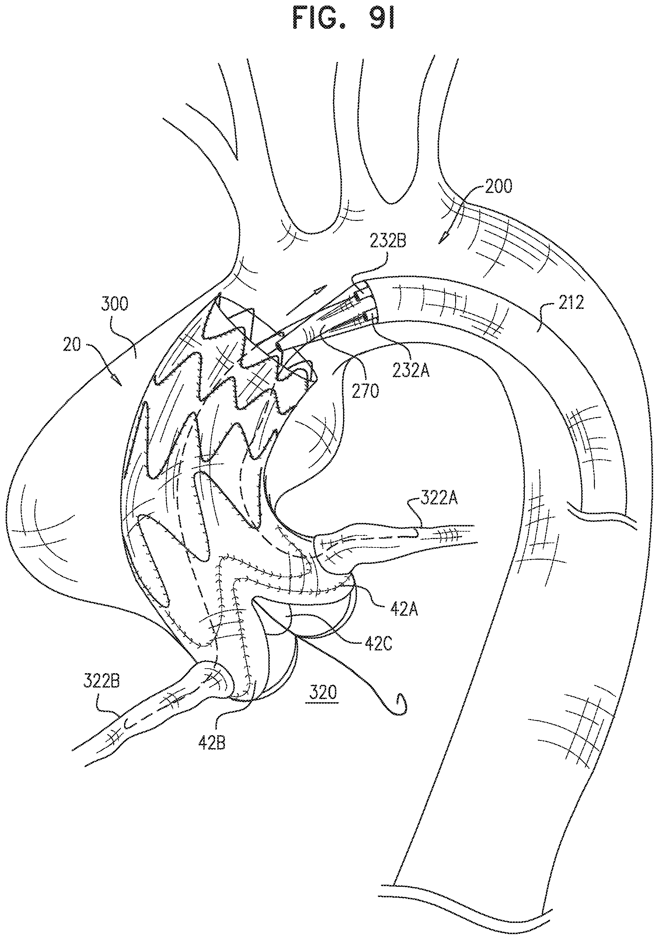

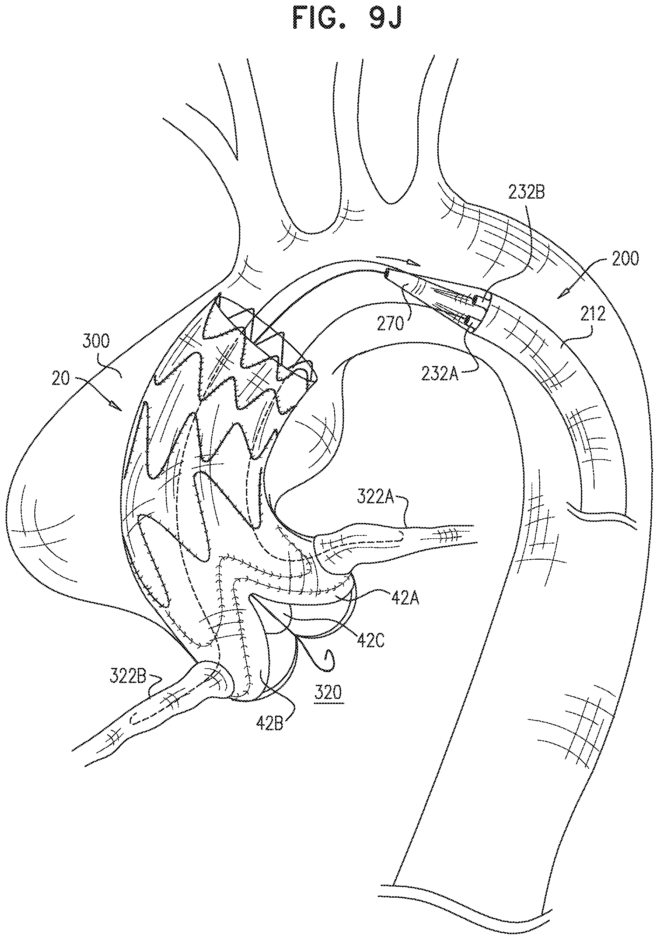

Reference is still made to Figs. 2A-C, and is additionally made to Fig. 3, which is a schematic illustration of a single proximally-extending piece 42, in accordance with an application of the present invention. When stent-graft 20 is unconstrained in its radially-expanded state, a proximal end portion 40 of covering element 32 is shaped so as to define at least first and second proximally-extending pieces 42A and 42B. The proximally-extending pieces are configured to be positioned at least partially in the aortic sinuses so as to provide a proximal landing zone for stent-graft 20, such as described hereinbelow with reference to Fig. 9F.

When stent-graft 20 is unconstrained in its radially-expanded state and proximally-extending pieces 42 are fully proximally extended, proximally-extending pieces 42 typically:
- are shaped so as to define respective distal bases 46, which (a) have respective base lengths L1 measured circumferentially around stent-graft 20, and (b) circumferentially circumscribe respective base arcs α (alpha), each of which has an angle of at least 100 degrees, no more than 140 degrees, and/or between 100 and 140 degrees;
- are shaped so as to define respective proximal-most portions 48, which are more proximal than all other respective portions of covering element 32 that circumscribe the respective base arcs α (alpha); in other words, each proximal-most portion 48 is at the most proximal location of its proximally-extending piece 42 (although another of the proximally-extending pieces may include a more proximal portion); and
- have respective axial lengths L2, measured axially between respective distal bases 46 and respective proximal-most portions 48, which lengths L2 equal at least 50%, no more than 150%, and/or between 50% and 150% of respective base lengths L1; for example lengths L2 may be at least 15 mm, no more than 30 mm, and/or between 15 mm and 30 mm.

For some applications, base lengths L1 of first and second proximally-extending pieces 42A and 42B are each at least 30 mm, no more than 50 mm, and/or between 30 and 50 mm. For some applications, when fully proximally extended, first and second proximally-extending pieces 42A and 42B have respective surface areas equal to at least 15% (such as at least 30%, no more than 150%, and/or between 15% (such as 30%) and 150% of the square of respective base lengths L1. For example, the surface area of each of the proximally-extending pieces may be equal to at least 100 mm2, no more than 400 mm2, and/or between 100 and 400 mm2. For some applications, an arc β (beta) (labeled in Fig. 2A) between circumferentially nearest portions of first and second proximally-extending pieces 42A and 42B has an angle of at least 5 degrees, no more than 50 degrees, and/or between 5 and 50 degrees, when stent-graft 20 is unconstrained in the radially-expanded state. Typically, first and second proximally-extending pieces 42A and 42B have substantially a same shape and size.

Reference is made to Figs. 1A-B, 2A, 2B, and 3. For some applications, first and second proximally-extending pieces 42A and 42B are shaped so as to define respective fenestrations 50 through covering element 32. Typically, each of fenestrations 50 has an area of at least 6 mm2, no more than 35 mm2, and/or between 6 and 35 mm2 when stent-graft 20 is unconstrained in the radially-expanded state and proximally-extending pieces 42 are fully proximally extended. First and second branching covered stents 22 (shown in Figs. 1A-B) have respective end portions 52 that are sized and configured to form blood-impervious seals with respective fenestrations 50. For some applications, each of fenestrations 50 is generally circular when the stent-graft is unconstrained in the radially-expanded state and the proximally-extending pieces are fully proximally extended. For example, a diameter of each of fenestrations 50 may be at least 3 mm, no more than 8 mm, and/or between 3 and 8 mm, when stent-graft 20 is unconstrained in the radially-expanded state and proximally-extending pieces 42 are fully proximally extended.

Typically, first proximally-extending piece 42A is attached to one or more of structural stent elements 31, and second proximally-extending piece 42B is attached to one or more of structural stent elements 31. For some applications, first and second proximally-extending pieces 42A and 42B are attached to the same one or more structural elements 31, such as shown in Figs. 1A-B and 2A-C; alternatively, they are attached to different ones of the structural elements (configuration not shown). For some applications, structural stent elements 31 are attached such that at least a portion of the one or more of the structural stent elements is proximal to fenestrations 50 when stent-graft 20 is unconstrained in the radially-expanded state and proximally-extending pieces 42 are fully proximally extended. For example, one or more of the structural stent elements may be attached along at least a portion of a proximal border of each of proximally-extending pieces 42A and 42B. These proximally-positioned structural stent elements may provide structure to the proximally-extending pieces, and may help anchor the proximally-extending pieces to the walls of the aortic sinuses. For some applications, fenestrations 50 are circumferentially centered on respective proximally-extending pieces 42 when stent-graft 20 is unconstrained in the radially-expanded state and proximally-extending pieces 42 are fully proximally extended.

For some applications, as labeled in Fig. 3, respective closest distances D2 of fenestrations 50 to proximal-most portions 48 equal at least 10%, no more than 30%, and/or between 10% and 30% of respective axial lengths L2, when stent-graft 20 is unconstrained in the radially-expanded state and proximally-extending pieces 42 are fully proximally extended.

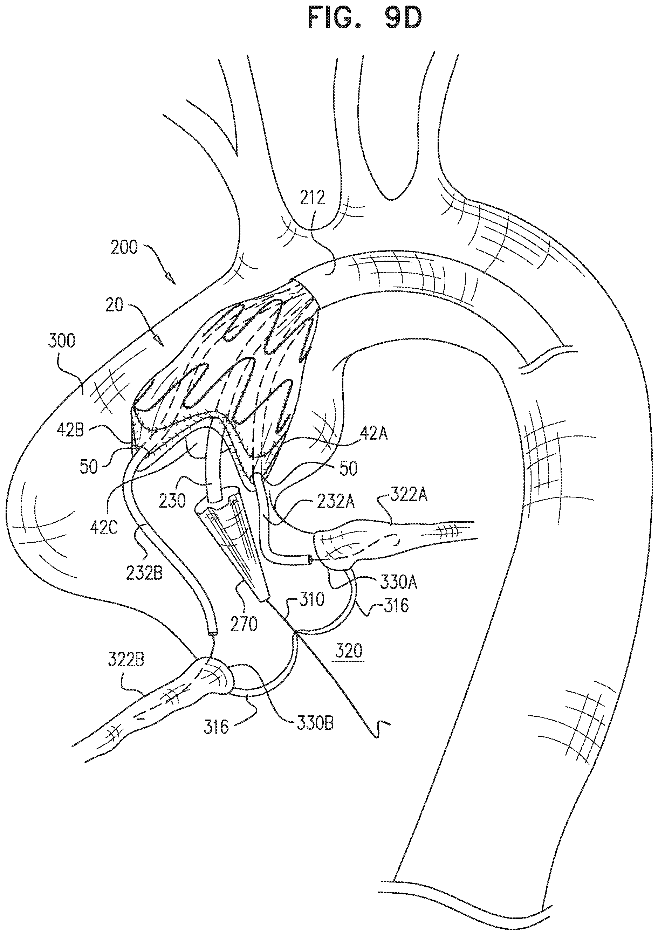

For some applications, stent-graft 20 further comprises at least one radiopaque wire 60 that is securely mounted around one of fenestrations 50, e.g., two radiopaque wires 60 that are securely mounted around respective fenestrations 50. Radiopaque wires 60 may facilitate proper positioning of fenestrations 50 with respect to the coronary ostia in the aortic sinuses, such as described hereinbelow with reference to Figs. 9D-E. In addition, the radiopaque wires may provide structural support to the borders of the fenestrations for good coupling with branching covered stents 22. For some applications, non-radiopaque wires are instead provided around the fenestrations for providing such coupling without the radiopacity.

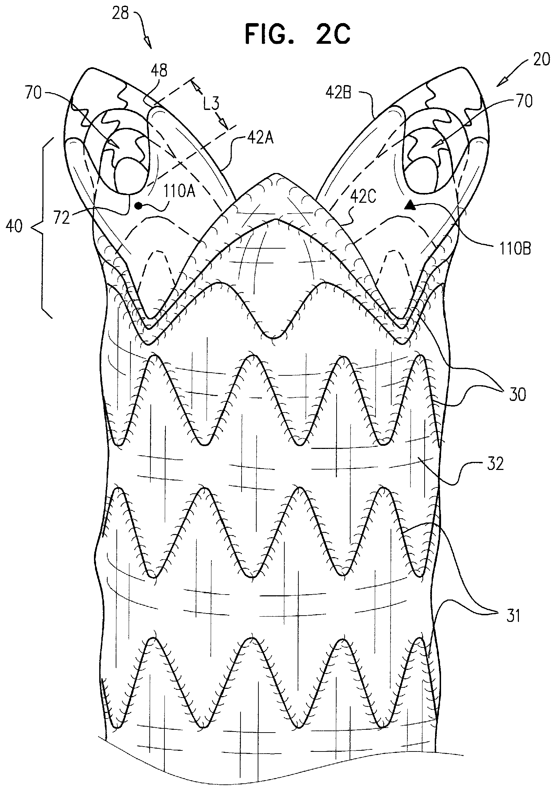

Reference is made to Fig. 2C. For some applications, first and the second proximally-extending pieces 42A and 42B are shaped so as to define respective scallops 70. Scallops 70 have respective axial lengths L3 measured from respective distal-most portions 72 of scallops 70 to respective proximal most-portions 48 of respective proximally-extending pieces 42, each of which axial lengths L3 is at least 7 mm, no more than 25 mm, and/or between 7 and 25 mm when stent-graft 20 is unconstrained in the radially-expanded state and proximally-extending pieces 42 are fully proximally extended.

For some applications, such as shown in Fig. 2C, one or more of structural stent elements 31 are attached to each of first and second proximally-extending pieces 42A and 42B such that respective portions of at least one of the one or more of structural stent elements 31 traverse scallops 70 when stent-graft 20 is unconstrained in the radially-expanded state and the proximally-extending pieces are fully proximally extended. These traversing portions may serve to provide structure to the proximally-extending pieces and/or to provide elements to which branching covered stents 22 are securely coupled. Alternatively or additionally, for some applications, such as shown in Fig. 2C, one or more of structural stent elements 31 are attached to each of first and second proximally-extending pieces 42A and 42B such that respective portions of at least one of the one or more of structural stent elements 31 are disposed proximal to proximal most-portions 48 of proximally-extending pieces. These proximally-extending portions may help anchor the proximally-extending pieces to the walls of the aortic sinuses.

Reference is again made to Figs. 1A-B, 2A, 2B, and 3. For some applications, first and second proximally-extending pieces 42A and 42B are shaped as first and second lobes 80 (labeled in Fig. 3), respectively, when stent-graft 20 is unconstrained in the radially-expanded state and proximally-extending pieces 42 are fully proximally extended. For some applications, first and second lobes 80 are semicircular, when the stent-graft is unconstrained in the radially-expanded state and the lobes are fully proximally extended; for example, each of first and second semicircular lobes 80 may be shaped so as to circumscribe approximately 180 degrees of a circle, between 100 and 180 degrees of a circle (e.g., between 100 and 170 degrees of a circle), or between 180 to 270 degrees of a circle (e.g., between 190 and 270 degrees of a circle), when the stent-graft is unconstrained in the radially-expanded state and the lobes are fully proximally extended.

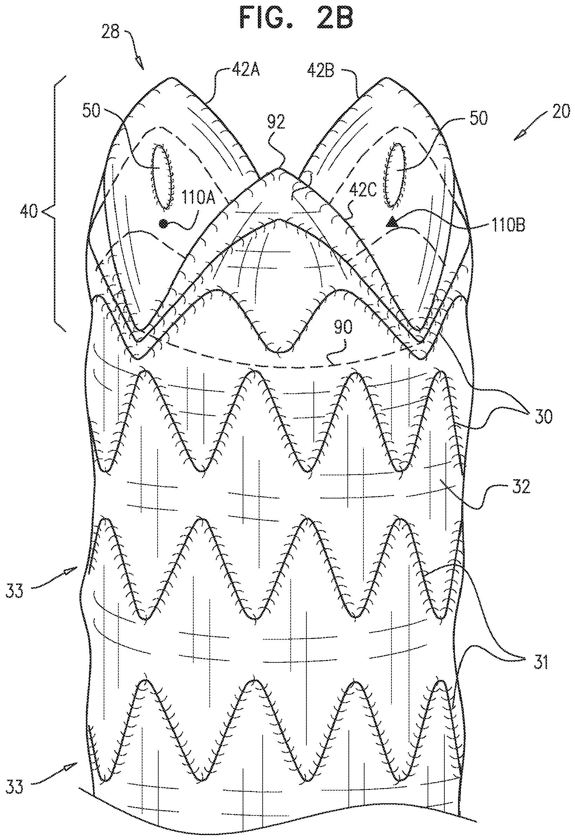

Reference is made to Figs. 1A-B and 2A-C. For some applications, when stent-graft 20 is unconstrained in the radially-expanded state, proximal end portion 40 of covering element 32 is shaped so as to define a third proximally-extending piece 42C. When fully proximally extended, third proximally-extending piece 42C:
- is shaped so as to define a third distal base 90 (labeled in Fig. 2B), which (a) has a third base length measured circumferentially around stent-graft 20, and (b) circumferentially circumscribes a third base arc, which has an angle of at least 100 degrees, no more than 140 degrees, and/or between 100 and 140 degrees;
- is shaped so as to define a third proximal-most portion 92, which is more proximal than all other portions of covering element 32 that circumscribe the third base arc; and
- has a third axial length, measured axially between third distal base 90 and third proximal-most portion 92, which length equals at least 50%, no more than 150%, and/or between 50% and 150% of the third base length.

Reference is made to Figs. 1A-B, 2A, 2B, and 3. For some applications in which first and second proximally-extending pieces 42A and 42B are shaped so as to define respective fenestrations 50, third proximally-extending piece 42C is not shaped so as to define any fenestrations through covering element 32. Reference is made to Fig. 2C. For some applications in which first and the second proximally-extending pieces 42A and 42B are shaped so as to define respective scallops 70, third proximally-extending piece 42C is not shaped so as to define any scallops.

For some applications, first, second, and third proximally-extending pieces 42A, 42B, and 42C have substantially a same shape and size, except that third proximally-extending piece 42C typically does not define a fenestration or scallop.

Typically, one or more of the structural stent elements 31 are attached to third proximally-extending piece 42C, which may provide structure to the proximally-extending piece.

Reference is made to Figs. 1A-B, 2A, and 2C. For some applications, as shown in these figures, proximally-extending pieces 42A, 42B, and/or 42C are outwardly convex (i.e., convex as viewed from outside the stent-graft) when stent-graft 20 is unconstrained in the radially-expanded state. Such convexity may facilitate good contact between the proximally-extending pieces and the walls of the aortic sinuses. For example, proximally-extending pieces 42A, 42B, and/or 42C may be outwardly conically flared at an angle γ (gamma) (labeled in Fig. 2A) with a central longitudinal axis 100 of stent-graft 20, when stent-graft 20 is unconstrained in the radially-expanded state and proximally-extending pieces 42 are fully proximally extended. Typically γ (gamma) is at least 10 degrees, no more than 30 degrees, and/or between 10 and 30 degrees.

Reference is made to Fig. 2B. For some applications, as shown in this figure, proximally-extending pieces 42A, 42B, and/or 42C are not outwardly convex, e.g., generally conform to the tubular geometry of stent-graft 20, when stent-graft 20 is unconstrained in the radially-expanded state.

Reference is made to Figs. 2A-C. For some applications, stent-graft 20 further comprises one or more radiopaque markers 110, which are securely mounted to stent-graft 20 to distinguish between first and second proximally-extending pieces 42A and 42B. For some applications, at least a first one 110A of radiopaque markers 110 is positioned on first proximally-extending piece 42A, and/or at least a second one 110B of radiopaque markers 110 is positioned on second proximally-extending piece 42B. Optionally, first and second radiopaque markers 110A and 110B have different respective shapes.

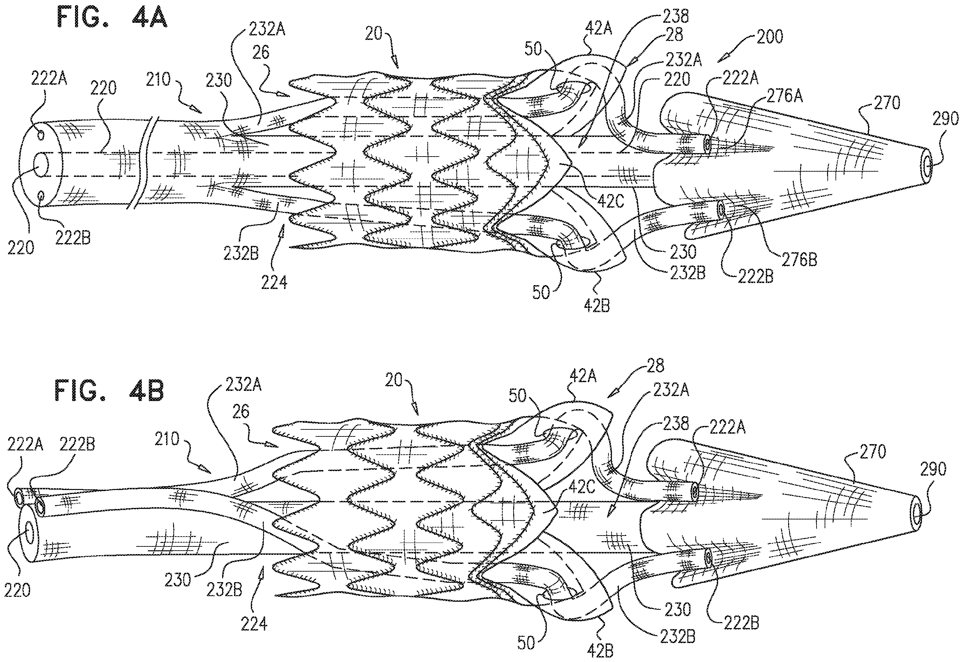

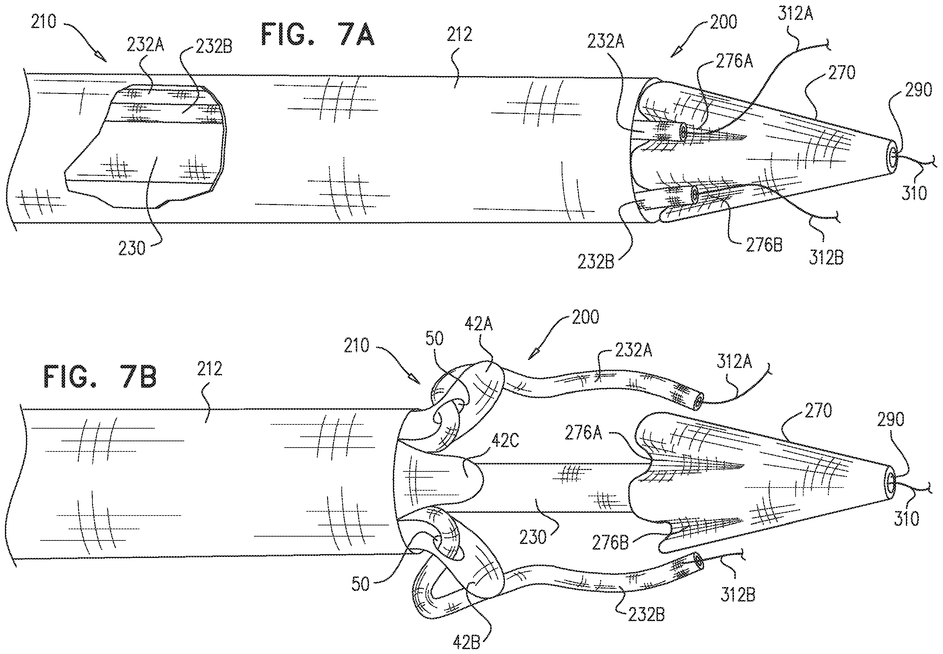

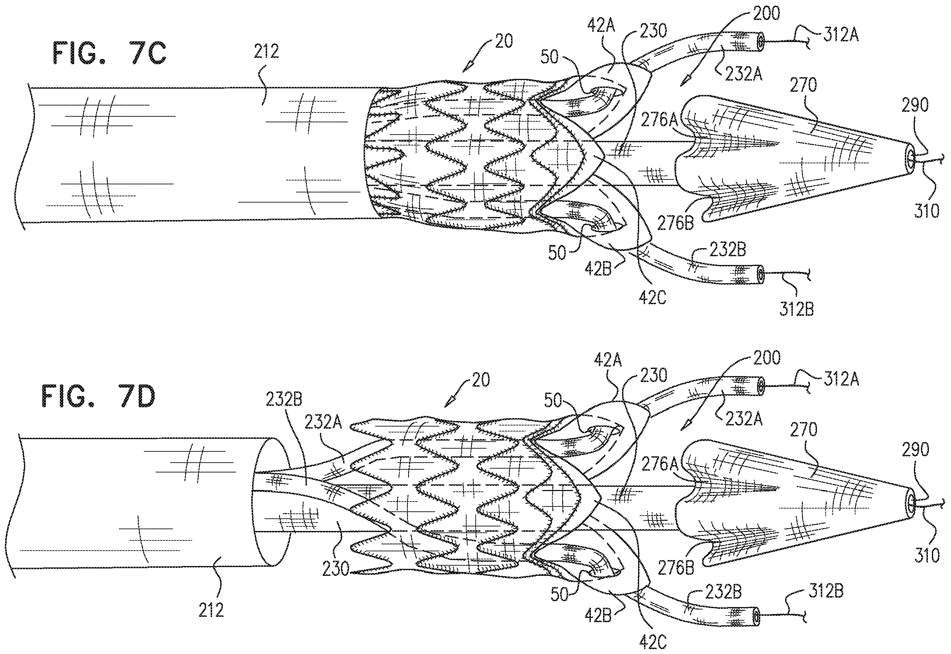

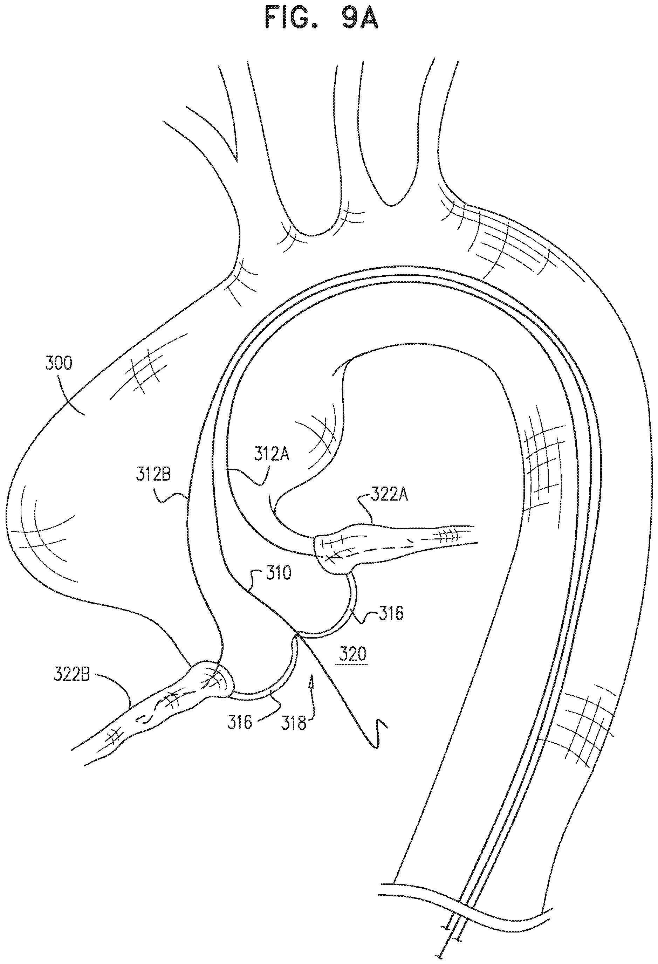

Reference is now made to Figs. 4A-B, which are schematic illustrations of components of a delivery tool, upon which stent-graft 20 is removably positioned, in accordance with respective applications of the present invention. Delivery tool 200 is configured to convey stent-graft 20 in the radially-compressed state to a target location in vasculature of a subject, and deploy the stent-graft at the target location, such as described hereinbelow with reference to Figs. 9A-J. Delivery tool 200 comprises at least one inner shaft 210 and an external sheath 212, described hereinbelow with reference to Figs. 7A-D. The at least one inner shaft 210 is shaped so as to define a primary bore 220 and first and second secondary bores 222A and 222B therethrough. For delivery, stent-graft 20 is removably positioned such that distal stent-graft end 26 surrounds an axial portion 224 of the at least one inner shaft 210. Stent-graft 20 is shown in Figs. 4A and 4B in the radially-expanded state.

For some applications, the at least one inner shaft 210 comprises (a) a primary inner shaft 230, which is shaped so as to define primary bore 220 therethrough, and (b) first and second secondary inner shafts 232A and 232B, which are shaped so as to define first and second secondary bores 222A and 222B therethrough, respectively. Typically, for delivery stent-graft 20 is removably positioned such that:
- distal stent-graft end 26 surrounds respective axial portions 224 of primary inner shaft 230 and first and second secondary inner shafts 232A and 232B;
- proximal stent-graft end 28 surrounds an axial portion 238 of primary inner shaft 230; and
- first and second secondary inner shafts 232A and 232B extend proximally beyond proximal stent-graft end 28.

For some applications, such as shown in Figs. 4A and 4B, in which first and second proximally-extending pieces 42A and 42B are shaped so as to define respective fenestrations 50, such as described hereinabove with reference to Figs. 1A-B, 2A, 2B, and 3, first and second secondary inner shafts 232A and 232B pass through first and second fenestrations 50, respectively, when stent-graft 20 is removably positioned such that first and second secondary inner shafts 232A and 232B extend proximally beyond proximal stent-graft end 28. When stent-graft 20 is in the radially-expanded state, a distal-to-proximal path along the secondary inner shafts passes from radially within to radially outside the proximally-extending pieces. This positioning of the secondary inner shafts enables the threading of secondary guidewires through fenestrations 50, as described hereinbelow with reference to Figs. 9A-B.

For some applications in which first and second proximally-extending pieces 42A and 42B are shaped so as to define respective scallops 70, such as described hereinabove with reference to Fig. 2C, first and second secondary inner shafts 232A and 232B pass through first and second scallops 70, respectively, when stent-graft 20 is removably positioned such that first and second secondary inner shafts 232A and 232B extend proximally beyond proximal stent-graft end 28 (configuration not shown). When stent-graft 20 is in the radially-expanded state, a distal-to-proximal path along the secondary inner shafts passes from radially within to radially outside the proximally-extending pieces. This positioning of the secondary inner shafts enables the threading of secondary guidewires through fenestrations 50, as described hereinbelow with reference to Figs. 9A-B.

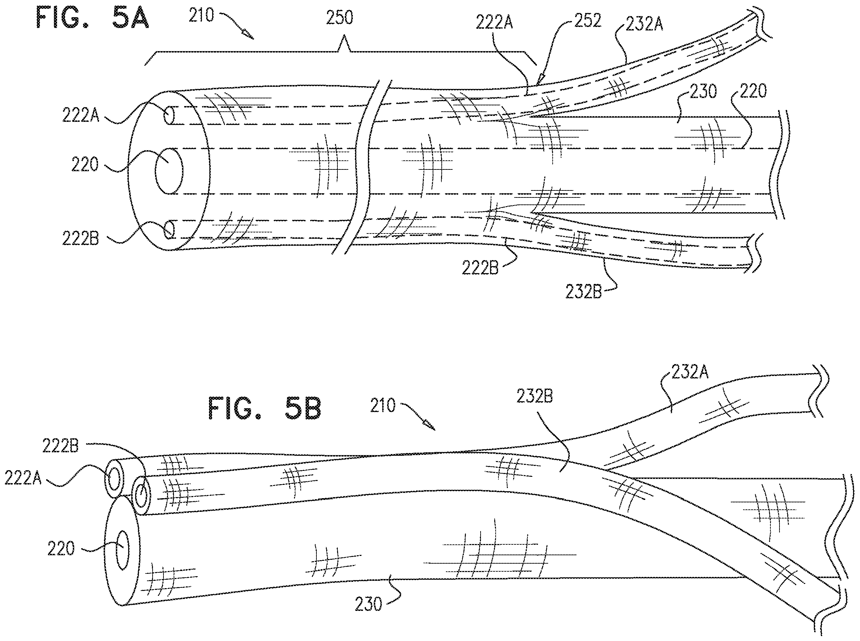

Reference is still made to Figs. 4A and 4B, and is additionally made to Figs. 5A and 5B, which are schematic illustrations of a portion of primary inner shaft 230 and first and second secondary inner shafts 232A and 232B, in accordance with respective applications of the present invention. The configurations shown in Figs. 5A and 5B correspond with the configurations shown in Figs. 4A and 4B, respectively.

In the configuration shown in Figs. 4A and 5A, a distal end portion 250 of the at least one inner shaft 210 is shaped so as to define primary bore 220 and first and second secondary bores 222A and 222B. At a proximal end 252 of distal end portion 250, the at least one inner shaft 210 trifurcates into primary inner shaft 230 and first and second secondary inner shafts 232A and 232B. In the configuration shown in Figs. 4B and 5B, the at least one inner shaft 210, along an entire length thereof, comprises separate primary inner shaft 230 and first and second secondary inner shafts 232A and 232B.

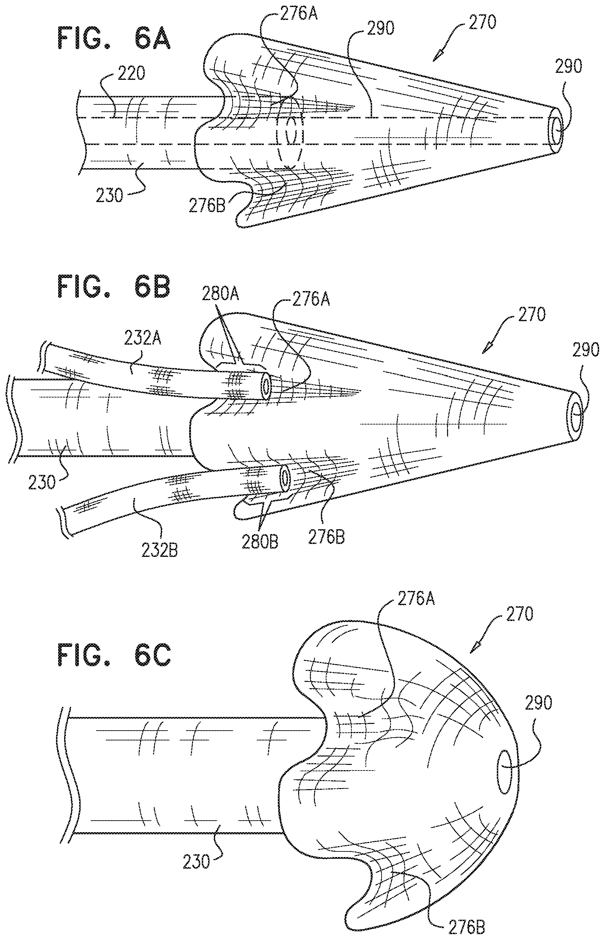

Reference is still made to Figs. 4A and 4B, and is additionally made to Figs. 6A, 6B, and 6C, which are schematic illustrations of a proximal tip 270 of delivery tool 200, in accordance with respective applications of the present invention. Proximal tip 270 is coupled to a proximal end portion of the at least one inner shaft 210, typically of primary inner shaft 230 when provided.

Proximal tip 270 is configured to reduce potential damage to a blood vessel wall when the delivery tool is proximally translated relative to the vessel wall. For some applications, such as shown in Figs. 4A-B and 6A-B, proximal tip 270 is conically shaped, such that the tip has a smallest cross-sectional area at a proximal-most portion of the tip. For other applications, such as shown in Fig. 6C, the proximal tip is hemispherically shaped, such that the tip has a smallest cross-sectional area at a proximal-most portion of the tip.

For some applications, an external surface 274 of proximal tip 270 is shaped so as to define first and second grooves 276A and 276B. The grooves extend axially along at least an axial portion 278 of tip 270, and are shaped and sized so as to reversibly receive proximal end portions 280A and 280B of first and second secondary inner shafts 232A and 232B, respectively, such that proximal end portions 280A and 280B are disposed in the respective grooves. Typically, the grooves reach a distal end of proximal tip 270. For some applications, the grooves taper from their distal ends toward their proximal ends. For some applications, at their largest portions, the grooves have (a) widths, measured circumferentially around the tip, approximately equal to an outer diameter of secondary inner shafts 232, and/or (b) depths equal to between 50% and 150% of the outer diameter of secondary inner shafts 232. For some applications, external surface 274 of proximal tip 270 is shaped so as to define exactly one groove 276, such as for applications in which only a single secondary inner shaft 232 is provided.

Typically, proximal tip 270 is shaped so as to define a tip bore 290 therethrough, and the tip bore and primary bore 220 are arranged axially continuously, such as shown in Fig. 6A.

Reference is now made to Figs. 7A-D, which are schematic illustrations of a portion of a deployment using delivery tool 200, in accordance with an application of the present invention. In Fig. 7A, stent-graft 20 is removably positioned, while in the radially-compressed state, within external sheath 212, such that distal stent-graft end 26 surrounds axial portion 224 of the at least one inner shaft 210, as shown in Figs. 4A-B. Typically, grooves 276A and 276B and first and second inner shafts 232A and 232B are sized and shaped such that proximal end portions 280A and 280B of first and second secondary inner shafts 232A and 232B extend radially no more than does an external surface of external sheath 212, so as to provide smooth proximal advancement of the delivery tool through the vasculature.

As shown in Figs. 7B-D, delivery tool 200 is configured such that axial translation of external sheath 212 (distally, to the left in Figs. 7B-D) facilitates a transition of stent-graft 20 from the radially-compressed state to the radially-expanded state. Typically, grooves 276 and secondary inner shafts 232 are sized and shaped such that the secondary inner shafts rest in the grooves loosely enough to passively disengage with external sheath 212 is withdrawn, as shown in Fig. 7B. Before withdrawal of external sheath 212, secondary inner shafts 232 are typically held in place in the grooves by external sheath 212, as shown in Fig. 7A.

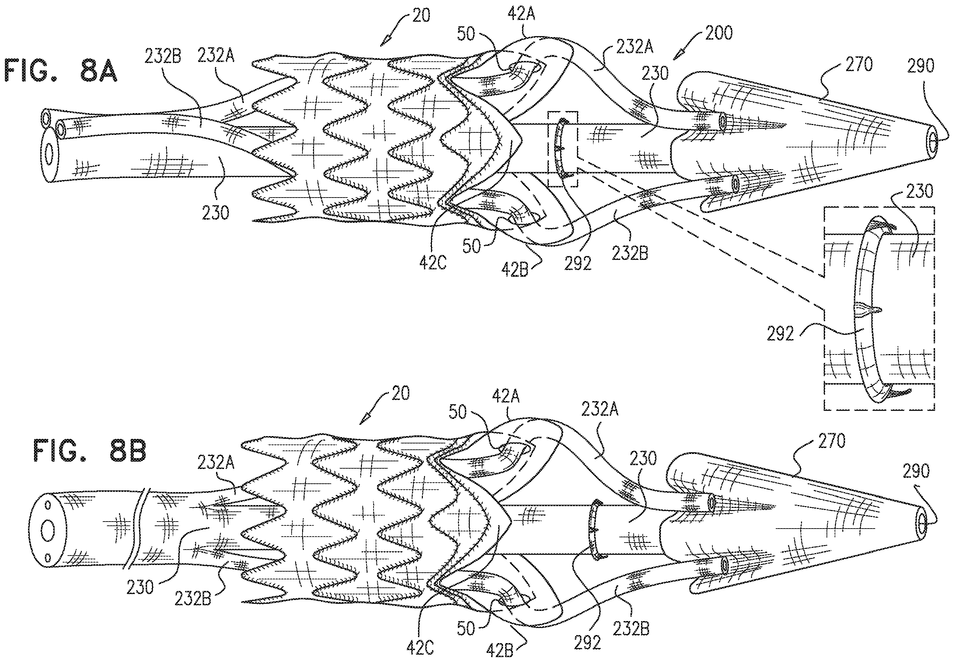

Reference is now made to Figs. 8A and 8B, which are schematic illustrations of additional configurations of delivery tool 200, in accordance with respective applications of the present invention. In these configurations, delivery tool 200 further comprises at least one stent-graft support member 292 that is securely fixed to an external surface of primary inner shaft 230, typically circumferentially disposed around the primary inner shaft. Stent-graft support member 292 is configured to prevent distal axial translation of stent-graft 20 as external sheath 212 is distally axially translated to facilitate the transition of stent-graft 20 from the radially-compressed state to the radially-expanded state, as described hereinabove with reference to Figs. 7A-D.

Typically, stent-graft support member 292 is positioned proximally adjacent to proximal stent-graft end 28, when stent-graft 20 is removably positioned, while in the radially-compressed state, within external sheath 212 with proximal stent-graft end 28 surrounding the axial portion of primary inner shaft 230.

Reference is now made to Figs. 9A-K, which are schematic illustrations of an exemplary method, not part of the invention, of deploying stent-graft 20 and two branching covered stents 22 in an ascending aorta 300, using delivery tool 200, in accordance with an application of the present invention. The aorta is aneurysmatic, or the aortic wall may suffer from a dissection. As used in the present application, including in the claims, a "lesion" of a blood vessel means an aneurysm and/or a dissection.

As shown in Fig. 9A, during a first stage of the implantation procedure, a primary guidewire 310 and two secondary guidewires 312A and 312B are endovascularly (typically percutaneously) introduced into the vasculature at a vascular access site, such as a femoral artery or an iliac artery. The guidewires are advanced to ascending aorta 300, typically via the descending aorta. Primary guidewire 310 is typically advanced between leaflets 316 of an aortic valve 318 into a left ventricle 320. Secondary guidewires 312A and 312B are advanced into left and right coronary arteries 322A and 322B, respectively. Typically, primary guidewire 310 has a diameter of between 0.635 mm (0.025") and 1.016 mm (0.04"), such as 0.889 mm (0.035"), and secondary guidewires 312 have a smaller diameter, such as between 0.254 mm (0.01") and 0.508 mm (0.02"), e.g., 0.3556 mm (0.014").

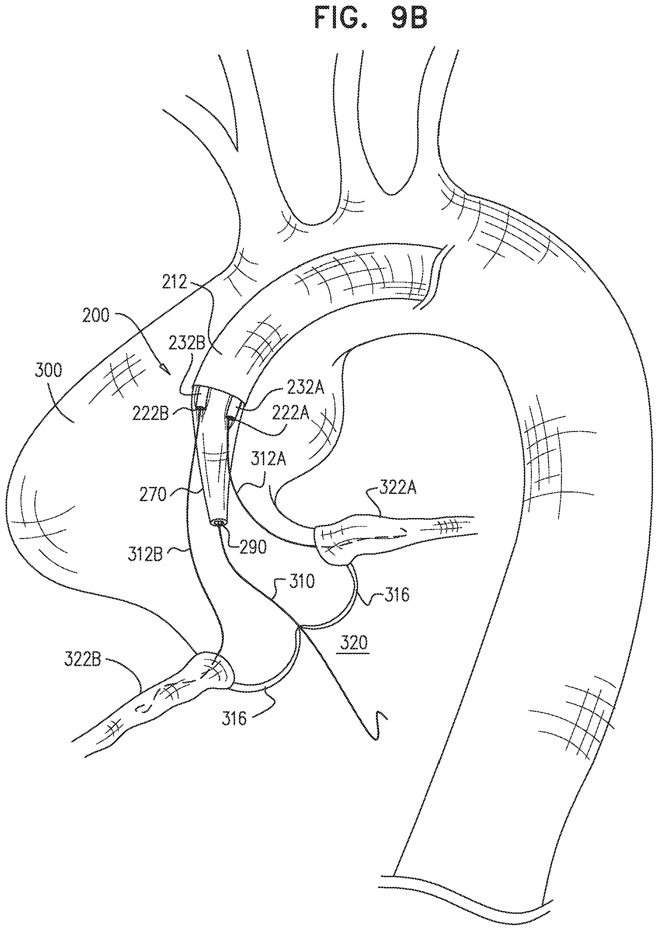

Outside the patient's body, a distal end of primary guidewire 310 is threaded through tip bore 290 and primary bore 220, and respective distal ends of secondary guidewires 312A and 312B are threaded through first and second secondary bores 222A and 222B, respectively. Typically, secondary inner shafts 232A and 232B are kink-resistant, so that the secondary guidewires can be readily threaded through the secondary bores despite the radial compression of stent-graft 20 and the secondary inner shafts in external sheath 212. As shown in Fig. 9B, delivery tool 200 is advanced over the three guidewires into ascending aorta 300, while stent-graft 20 is removably positioned, while in the radially-compressed state, within external sheath 212 (stent-graft 20 is not visible in the sheath in Fig. 9B).

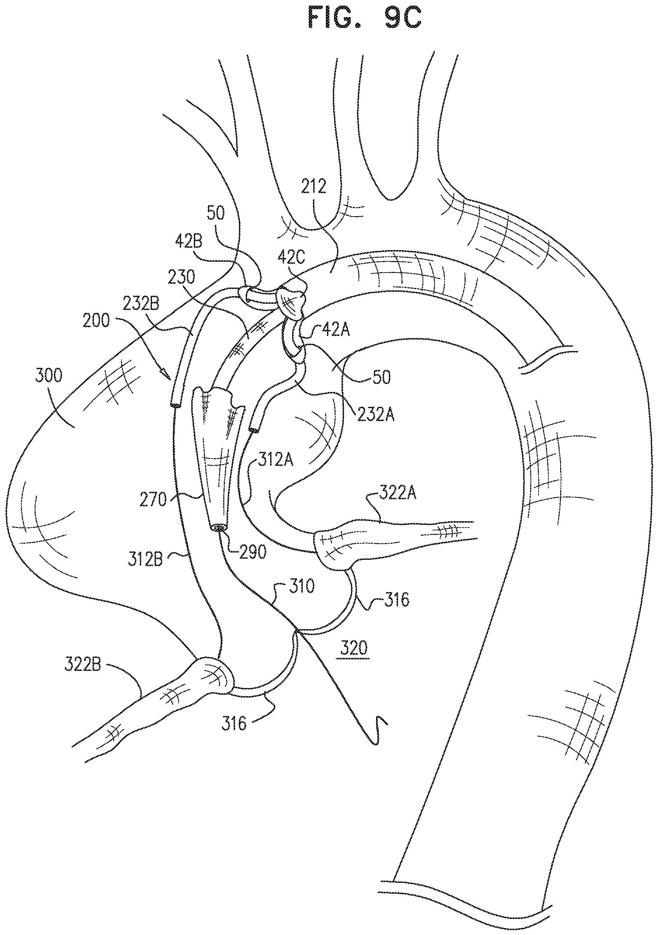

As shown in Figs. 9C and 9D, external sheath 212 is distally axially translated, so as to facilitate a partial transition of stent-graft 20 from the radially-compressed state to the radially-expanded state. As can be seen in these figures, first and second secondary inner shafts 232A and 232B pass through first and second fenestrations 50, as do secondary guidewires 312A and 312B, which pass through the secondary inner shafts, respectively. Stent-graft support member 292, if provided, prevents distal axial translation of stent-graft 20 as external sheath 212 is distally translated.

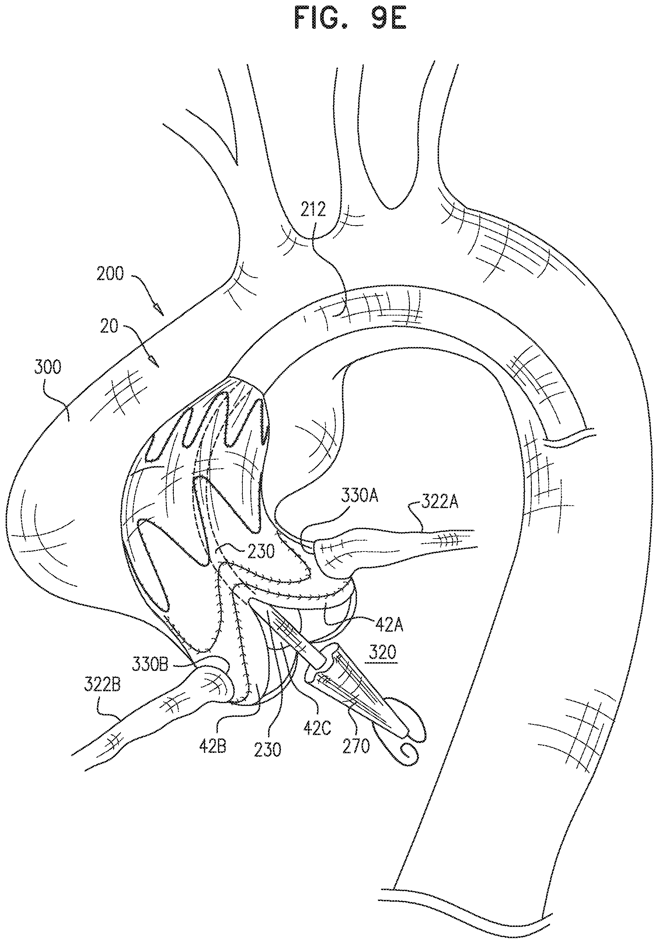

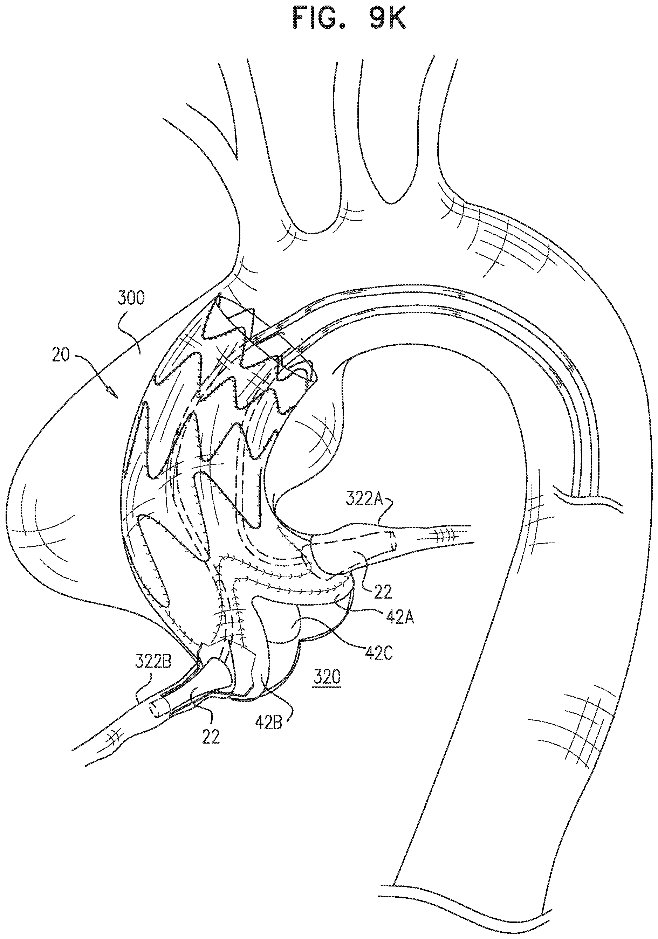

As shown in Figs. 9D and 9E, secondary guidewires 312A and 312B guide respective fenestrations 50 of proximally-extending pieces 42A and 42B to respective left and right coronary ostia 330A and 330B, and align the fenestrations with the ostia. Such alignment facilitates the deployment of branching covered stents 22 in coronary arteries 322A and 322B, as described hereinbelow with reference to Fig. 9K. Proximal tip 270 may be advanced through leaflets 316 of aortic valve 318 into left ventricle 320, which may improve the stabilization of the delivery system while deploying the stent grafts and covered branching stents.

For configurations of stent-graft 20 in which proximally-extending pieces 42 are shaped so as to define scallops 70, such as described hereinabove with reference to Fig. 2C, secondary guidewires 312A and 312B guide respective scallops 70 of proximally-extending pieces 42A and 42B to respective left and right coronary ostia 330A and 330B, and align the scallops with the ostia.

As shown in Fig. 9F, external sheath 212 is further distally axially translated, so as to facilitate the remainder of the transition of stent-graft 20 from the radially-compressed state to the radially-expanded state. As a result of the deployment, proximally-extending pieces 42A and 42B, as well as third proximally-extending piece 42C, if provided, are positioned at least partially in aortic sinuses 332, respectively, so as to provide a proximal landing zone for stent-graft 20. A relatively long landing zone of blood vessel wall, such as about 3 cm, is desirable to provide good anchoring and sealing in the ascending aorta, because the ascending aorta is highly motile and pulsatile. Proximal-most portions 48 of proximally-extending pieces 42A and 42B, and of third proximally-extending piece 42C if provided, are positioned in aortic sinuses 332, respectively. Proximal-most portions 48 of proximally-extending pieces 42A and 42B may touch the sinus floors at respective bases of leaflets 316. Fenestrations 50 are aligned with coronary ostia 330, or for configurations of stent-graft 20 in which proximally-extending pieces 42 are shaped so as to define scallops 70, such as described hereinabove with reference to Fig. 2C, the scallops are aligned with coronary ostia 330.

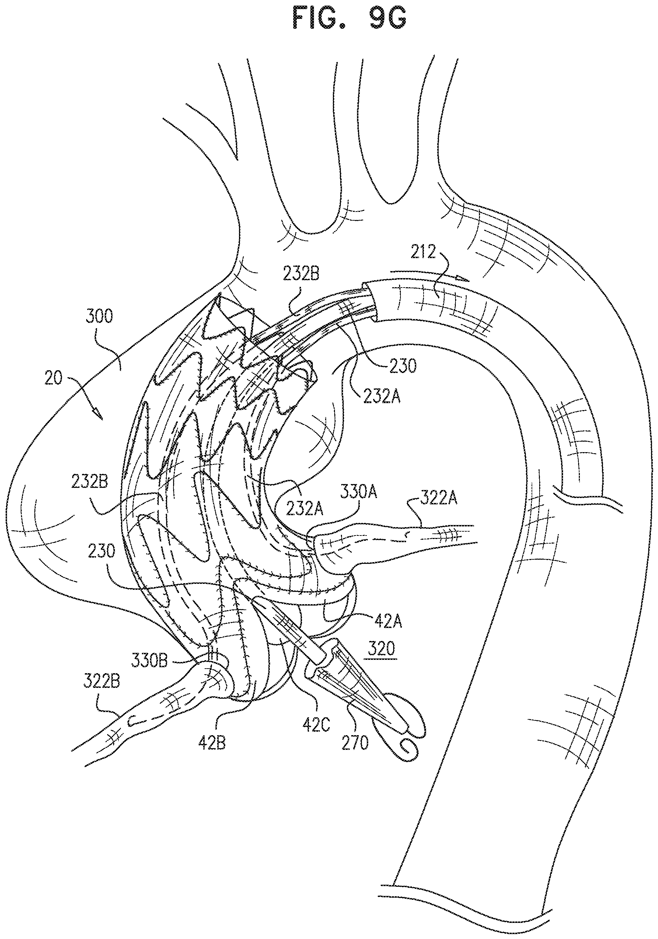

As shown in Figs. 9G-J, delivery tool 200 is withdrawn from ascending aorta 300 and the patient's vasculature, typically leaving at least secondary guidewires 312A and 312B in place, extending into the coronary arteries.

As shown in Fig. 9K, branching covered stents 22 are deployed, typically over secondary guidewires 312A and 312B, into coronary arteries 322A and 322B. Branching covered stents 22 are coupled to stent-graft 20 so as to form blood-impervious seals with respective fenestrations 50. The coupling may be performed using stent-graft coupling techniques known in the art and/or described in PCT Publications WO 2011/007354, WO 2011/064782, and/or WO 2013/005207, all of which are assigned to the assignee of the present application. For configurations of stent-graft 20 in which proximally-extending pieces 42 are shaped so as to define scallops 70, such as described hereinabove with reference to Fig. 2C, branching covered stents 22 are coupled to stent-graft 20 so as to form blood-impervious seals with the partial borders of the scallops, respectively.

Fig. 9K shows stent-graft 20 fully implanted in ascending aorta 300 and branching covered stents 22 fully implanted in left and right coronary arteries 322A and 322B. Stent-graft 20 and branching covered stents 22 together provide blood-flow paths (a) through ascending aorta 300, bypassing the aneurysm or the dissection, and (b) to left and right coronary arteries 322A and 322B. The coronary arteries typically provide stability to the branching covered stents.

As used in the present application, including in the claims, a "fenestration" is an opening entirely surrounded by a covering element. For example, scallops 70, described hereinabove with reference to Fig. 2C, are not fenestrations, because the scallops are open to the proximal ends of the proximally-extending pieces and thus are not surrounded by the covering element.

As used in the present application, including in the claims, "tubular" means having the form of an elongated hollow object that defines a conduit therethrough. A "tubular" structure may have varied cross-sections therealong, and the cross-sections are not necessarily circular. For example, one or more of the cross-sections may be generally circular, or generally elliptical but not circular, or circular.

Although the stent-grafts and delivery tool 200 has been described herein as being deployed in the ascending aorta, for some applications they are instead deployed in another blood vessel, such as in the descending aorta, e.g., in the descending aorta and the branching renal arteries. For these applications, the stent-graft does not necessarily define the proximally-extending pieces. It is noted that in these applications, as well as the other applications described herein, first and second secondary inner shafts 232A and 232B typically pass through first and second fenestrations 50 while stent-graft 20 is radially compressed within external sheath 212. It is also noted that in these applications, as well as the other applications described herein, first and second secondary inner shafts 232A and 232B typically do not contain any stent-grafts.

The scope of the present invention includes embodiments described in the following applications, which are assigned to the assignee of the present application. In an embodiment, techniques and apparatus described in one or more of the following applications are combined with techniques and apparatus described herein:
- US Application 12/529,936, filed September 4, 2009, which issued as US Patent 8,317,856
- US Provisional Application 60/892,885, filed March 5, 2007
- US Provisional Application 60/991,726, filed December 2, 2007
- US Provisional Application 61/219,758, filed June 23, 2009
- US Provisional Application 61/221,074, filed June 28, 2009
- US Application 13/380,278, filed December 22, 2011, which published as US Patent Application Publication 2012/0150274
- US Application 13/384,075, filed January 13, 2012, which published as US Patent Application Publication 2012/0179236
- US Application 13/505,996, filed May 3, 2012, which published as US Patent Application Publication 2012/0310324
- US Application 13/512,778, filed September 24, 2012, which published as US Patent Application Publication 2013/0013050
- US Application 13/513,397, filed June 1, 2012, which published as US Patent Application Publication 2012/0330399
- US Application 13/514,240, filed June 6, 2012, which published as US Patent Application Publication 2013/0013051
- US Application 13/577,161, filed August 3, 2012, which published as US Patent Application Publication 2013/0035751
- US Application 13/031,871, filed February 22, 2011, which published as US Patent Application Publication 2011/0208289
- US Provisional Application 61/496,613, filed June 14, 2011
- US Provisional Application 61/505,132, filed July 7, 2011
- US Provisional Application 61/529,931, filed September 1, 2011
- PCT Application PCT/IL2012/000060, filed February 2, 2012, which published as PCT Publication WO 2012/104842
- PCT Application PCT/IL2012/000083, filed February 16, 2012, which published as PCT Publication WO 2012/111006
- PCT Application PCT/IL2012/000095, filed March 1, 2012, which published as PCT Publication WO 2012/117395
- PCT Application PCT/IL2012/000148, filed April 4, 2012, which published as PCT Publication WO 2013/030818
- PCT Application PCT/IL2012/000190, filed May 15, 2012, which published as PCT Publication WO 2013/171730
- US Patent Application 13/523,296, filed June 14, 2012, which published as US Patent Application Publication 2012/0323305
- PCT Application PCT/IL2012/000241, filed June 19, 2012, which published as PCT Publication WO 2012/176187
- PCT Application PCT/IL2012/000269, filed July 2, 2012, which published as PCT Publication WO 2013/005207
- PCT Application PCT/IL2012/050424, filed October 29, 2012, which published as PCT Publication WO 2013/065040
- PCT Application PCT/IL2012/050506, filed December 4, 2012, which published as PCT Publication WO 2013/084235
- US Provisional Application 61/749,965, filed January 8, 2013, entitled, "Minimization of stent-graft migration during implantation"
- US Provisional Application 61/775,964, filed March 11, 2013, entitled, "Multi-component stent-graft system for aortic dissections"

It will be appreciated by persons skilled in the art that the present invention is not limited to what has been particularly shown and described hereinabove. Rather, the scope of the present invention includes both combinations and subcombinations of the various features described hereinabove, as well as variations and modifications thereof that are not in the prior art, which would occur to persons skilled in the art upon reading the foregoing description.

## Claims

1. Apparatus comprising a generally tubular stent-graft (20), which has distal and proximal stent-graft ends (26, 28) and comprises:
a generally tubular support element (30), which comprises a plurality of structural stent elements (31); and
a covering element (32) that is attached to and at least partially covers the support element (30),
wherein when the stent-graft (20) is unconstrained in a radially-expanded state, a proximal end portion (40) of the covering element (32) is shaped so as to define at least first and second proximally-extending pieces (42A, 42B),
wherein when the stent-graft (20) is unconstrained in the radially-expanded state and the proximally-extending pieces (42A, 42B) are fully proximally extended, the proximally-extending pieces (42A, 42B):
are shaped so as to define respective distal bases (46), which (a) have respective base lengths (L1) measured circumferentially around the stent-graft (20), and (b) circumferentially circumscribe respective base arcs (α), each of which base arcs (α) has an angle of between 100 and 140 degrees,
are shaped so as to define respective proximal-most portions (48), which are more proximal than all other respective portions of the covering element (32) that circumscribe the respective base arcs (α), and
have respective axial lengths (L2), measured axially between the respective distal bases (46) and the respective proximal-most portions (48), which lengths (L2) equal between 50% and 150% of the respective base lengths (L1), and
wherein when the stent-graft (20) is unconstrained in the radially-expanded state and the proximally-extending pieces (42A, 42B) are fully proximally extended, the first and the second proximally-extending pieces (42A, 42B) are shaped so as to define first and second fenestrations (50), respectively, through the covering element (32), each of which fenestrations (50) has an area of at least 6 mm2,
wherein the apparatus further comprises a delivery tool (200), which (a) is configured to convey the stent-graft (20) in the radially-compressed state to a target location in vasculature of a subject, and deploy the stent-graft (20) at the target location, and (b) comprises:
a primary inner shaft (230), which is shaped so as to define a primary bore (220);
first and second secondary inner shafts (232A, 232B), which are shaped so as to define first and the second secondary bores (222A, 222B) therethrough, respectively; and an external sheath (212), and
wherein the delivery tool (200) is configured such that axial translation of the external sheath (212) facilitates a transition of the stent-graft (20) from the radially-compressed state to the radially-expanded state,
**characterized in that** the stent-graft (20) is removably positioned, while in the radially-compressed state, within the external sheath (212), such that:
(a) the distal stent-graft end (26) surrounds respective axial portions (224) of the primary inner shaft (230) and the first and the second secondary inner shafts (232A, 232B),
(b) the proximal stent-graft end (28) surrounds an axial portion (238) of the primary inner shaft (230), and
(c) the first and the second secondary inner shafts (232A, 232B) extend proximally beyond the proximal stent-graft end (28) and pass through the first and the second fenestrations (50), respectively.

2. The apparatus according to claim 1, wherein the proximally-extending pieces (42A, 42B) are outwardly conically flared at an angle of between 10 and 30 degrees with a central longitudinal axis of the stent-graft (20), when the stent-graft (20) is unconstrained in the radially-expanded state and the proximally-extending pieces (42A, 42B) are fully proximally extended.

3. The apparatus according to any one of claims 1-2, comprising a multi-component stent-graft system (10), which comprises:
the stent-graft (20); and
first and second branching covered stents (22), which have respective end portions (52) that are sized and configured to form blood-impervious seals with the first and the second fenestrations (50) of the first and the second proximally-extending pieces (42A, 42B) of the stent-graft (20), respectively.

4. The apparatus according to any one of claims 1-2, wherein each of the first and the second fenestrations (50) has an area of no more than 35 mm2 when the stent-graft (20) is unconstrained in the radially-expanded state and the proximally-extending pieces (42A, 42B) are fully proximally extended.

5. The apparatus according to any one of claims 1-2, wherein the first and the second proximally-extending pieces (42A, 42B) are shaped as first and second lobes, respectively, when the stent-graft (20) is unconstrained in the radially-expanded state and the proximally-extending pieces (42A, 42B) are fully proximally extended.

6. The apparatus according to any one of claims 1-2,
wherein when the stent-graft (20) is unconstrained in the radially-expanded state, the proximal end portion (40) of the covering element (32) is shaped so as to define a third proximally-extending piece (42C), and
wherein when the stent-graft (20) is unconstrained in the radially-expanded state and the third proximally-extending piece (42C) is fully proximally extended, the third proximally-extending piece (42C):
is shaped so as to define a third distal base (90), which (a) has a third base length measured circumferentially around the stent-graft (20), and (b) circumferentially circumscribes a third base arc, which third base arc has an angle of between 100 and 140 degrees,
is shaped so as to define a third proximal-most portion (92), which is more proximal than all other portions of the covering element (32) that circumscribe the third base arc, and
has a third axial length, measured axially between the third distal base (90) and the third proximal-most portion (92), which length equals between 50% and 150% of the third base length.

7. The apparatus according to claim 6, wherein the third proximally-extending piece (42C) is not shaped so as to define any fenestrations through the covering element (32).

8. The apparatus according to any one of claims 1-2,
wherein the delivery tool (200) further comprises a proximal tip (270) coupled to a proximal end portion of the primary inner shaft (230), and
wherein an external surface of the proximal tip (270) is shaped so as to define first and second grooves (276A, 276B), which (a) extend axially along at least an axial portion of the tip (270), and (b) are shaped and sized so as to reversibly receive respective proximal end portions (280A, 280B) of the first and the second secondary inner shafts (232A, 232B).

9. The apparatus according to any one of claims 1-2, wherein the delivery tool (200) further comprises at least one stent-graft support member (292) which is securely fixed to an external surface of the primary inner shaft (230), and which is configured to prevent distal axial translation of the stent-graft (20) as the external sheath (212) is distally axially translated to facilitate the transition of the stent-graft (20) from the radially-compressed state to the radially-expanded state.

10. The apparatus according to claim 9, wherein the stent-graft support member (292) is circumferentially disposed around the primary inner shaft (230).

11. The apparatus according to claim 9, wherein the stent-graft support member (292) is positioned proximally adjacent to the proximal stent-graft end (28), when the stent-graft (20) is removably positioned, while in the radially-compressed state, within the external sheath (212) with the proximal stent-graft end (28) surrounding the axial portion (238) of the primary inner shaft (230).

12. The apparatus according to any one of claims 1-2, wherein the delivery tool (200) further comprises a proximal tip (270) coupled to a proximal end portion of the primary inner shaft (230).

13. The apparatus according to claim 12, wherein the proximal tip (270) is conically shaped, such that the tip (270) has a smallest cross-sectional area at a proximal-most portion of the tip (270).

14. The apparatus according to claim 12, wherein the proximal tip (270) is hemispherically shaped, such that the tip (270) has a smallest cross-sectional area at a proximal-most portion of the tip (270).

## Patentansprüche

1. Vorrichtung, die ein generell röhrenförmiges Stenttransplantat (20) beinhaltet, das ein distales und proximales Stenttransplantatende (26, 28) aufweist und Folgendes beinhaltet:
ein generell röhrenförmiges Stützelement (30), das eine Vielzahl von strukturellen Stentelementen (31) beinhaltet; und
ein Abdeckelement (32), das am Stützelement (30) angebracht ist und dieses mindestens teilweise abdeckt,
wobei, wenn das Stenttransplantat (20) in einem radial expandierten Zustand ungebunden ist, ein proximaler Endabschnitt (40) des Abdeckelements (32) so geformt ist, dass mindestens ein erstes und zweites sich proximal erstreckendes Stück (42A, 42B) definiert wird,
wobei, wenn das Stenttransplantat (20) im radial expandierten Zustand ungebunden ist und die sich proximal erstreckenden Stücke (42A, 42B) vollständig proximal erstreckt sind, die sich proximal erstreckenden Stücke (42A, 42B):
so geformt sind, dass jeweilige distale Basen (46) definiert werden, die (a) jeweilige Basislängen (L1) aufweisen, umlaufend um das Stenttransplantat (20) gemessen, und (b) umlaufend jeweilige Basisbögen (α) abgrenzen, wobei jeder Basisbogen (α) einen Winkel zwischen 100 und 140 Grad aufweist,
so geformt sind, dass jeweilige proximalste Abschnitte (48) definiert werden, die proximaler sind als alle anderen jeweiligen Abschnitte des Abdeckelements (32), die die jeweiligen Basisbögen (α) abgrenzen, und
jeweilige Axiallängen (L2) aufweisen, axial zwischen den jeweiligen distalen Basen (46) und den jeweiligen proximalsten Abschnitten (48) gemessen, wobei die Längen (L2) zwischen 50 % und 150 % den jeweiligen Basislängen (L1) gleichen, und
wobei, wenn das Stenttransplantat (20) im radial expandierten Zustand ungebunden ist und die sich proximal erstreckenden Stücke (42A, 42B) vollständig proximal erstreckt sind, das erste und zweite sich proximal erstreckende Stück (42A, 42B) so geformt sind, dass eine erste und zweite Fenestration (50) durch das Abdeckelement (32) definiert wird, wobei jede Fenestration (50) eine Fläche von mindestens 6 mm² aufweist,
wobei die Vorrichtung ferner ein Zustellwerkzeug (200) beinhaltet, das (a) konfiguriert ist, um das Stenttransplantat (20) im radial komprimierten Zustand an eine Zielstelle in der Vaskulatur eines Subjekts zu befördern und das Stenttransplantat (20) an der Zielstelle zu entfalten, und (b) Folgendes beinhaltet:
einen primären Innenschaft (230), der so geformt ist, dass eine primäre Bohrung (220) definiert wird;
einen ersten und zweiten, sekundären Innenschaft (232A, 232B), die so geformt sind, dass eine erste bzw. zweite, sekundäre Bohrung (222A, 222B) durch diese definiert wird; und
eine externe Umhüllung (212), und
wobei das Zustellwerkzeug (200) konfiguriert ist, sodass axiale Translation der externen Umhüllung (212) einen Übergang des Stenttransplantats (20) vom radial komprimierten Zustand zum radial expandierten Zustand erleichtert,
**dadurch gekennzeichnet, dass** das Stenttransplantat (20) innerhalb der externen Umhüllung (212) entfernbar positioniert ist, während es im radial komprimierten Zustand ist, sodass:
(a) das distale Stenttransplantatende (26) jeweilige axiale Abschnitte (224) des primären Innenschafts (230) und des ersten und des zweiten sekundären Innenschafts (232A, 232B) umgibt,
(b) das proximale Stenttransplantatende (28) einen axialen Abschnitt (238) des primären Innenschafts (230) umgibt und
(c) sich der erste und der zweite sekundäre Innenschaft (232A, 232B) proximal über das proximale Stenttransplantatende (28) hinaus erstrecken und durch die erste bzw. die zweite Fenestration (50) hindurch gehen.

2. Vorrichtung gemäß Anspruch 1, wobei die sich proximal erstreckenden Stücke (42A, 42B) in einem Winkel zwischen 10 und 30 Grad zu einer zentralen Längsachse (20) nach außen konisch aufgeweitet sind, wenn das Stenttransplantat (20) im radial expandierten Zustand ungebunden ist und die sich proximal erstreckenden Stücke (42A, 42B) vollständig proximal erstreckt werden.

3. Vorrichtung gemäß einem der Ansprüche 1-2, die ein Mehrkomponenten-Stenttransplantatsystem (10) beinhaltet, das Folgendes beinhaltet:
das Stenttransplantat (20); und
einen ersten und zweiten verzweigten, abgedeckten Stent (22), die jeweilige Endabschnitte (52) aufweisen, die bemessen und konfiguriert sind, um blutundurchlässige Dichtungen mit der ersten und der zweiten Fenestration (50) des ersten bzw. des zweiten sich proximal erstreckenden Stückes (42A, 42B) des Stenttransplantats (20) zu bilden.

4. Vorrichtung gemäß einem der Ansprüche 1-2, wobei jede der ersten und der zweiten Fenestration (50) eine Fläche von nicht mehr als 35 mm² aufweist, wenn das Stenttransplantat (20) im radial expandierten Zustand ungebunden ist und die sich proximal erstreckenden Stücke (42A, 42B) vollständig proximal erstreckt sind.

5. Vorrichtung gemäß einem der Ansprüche 1-2, wobei das erste und das zweite sich proximal erstreckende Stück (42A, 42B) als erster bzw. zweiter Lappen geformt sind, wenn das Stenttransplantat (20) im radial expandierten Zustand ungebunden ist und die sich proximal erstreckenden Stücke (42A, 42B) vollständig proximal erstreckt sind.

6. Vorrichtung gemäß einem der Ansprüche 1-2,
wobei, wenn das Stenttransplantat (20) im radial expandierten Zustand ungebunden ist, der proximale Endabschnitt (40) des Abdeckelements (32) so geformt ist, dass ein drittes sich proximal erstreckende Stück (42C) definiert wird, und
wobei, wenn das Stenttransplantat (20) im radial expandierten Zustand ungebunden ist und das dritte sich proximal erstreckende Stück (42C) vollständig proximal erstreckt ist, das dritte sich proximal erstreckende Stück (42C):
so geformt ist, dass eine dritte distale Basis (90) definiert wird, die (a) eine dritte Basislänge aufweist, umlaufend um das Stenttransplantat (20) gemessen, und (b) umlaufend einen dritten Basisbogen abgrenzt, wobei der dritte Basisbogen einen Winkel zwischen 100 und 140 Grad aufweist,
so geformt ist, dass ein dritter proximalster Abschnitt (92) definiert wird, der proximaler ist als alle anderen jeweiligen Abschnitte des Abdeckelements (32), die den dritten Basisbogen abgrenzen, und
eine dritte Axiallänge aufweist, axial zwischen der dritten distalen Basis (90) und dem dritten proximalsten Abschnitt (92) gemessen, wobei die Länge zwischen 50 % und 150 % der dritten Basislänge gleicht.

7. Vorrichtung gemäß Anspruch 6, wobei das dritte sich proximal erstreckende Stück (42C) nicht so geformt ist, dass Fenestrationen durch das Abdeckelement (32) definiert werden.

8. Vorrichtung gemäß einem der Ansprüche 1-2,
wobei das Zustellwerkzeug (200) ferner eine proximale Spitze (270) beinhaltet, die mit einem proximalen Endabschnitt des primären Innenschafts (230) gekoppelt ist, und
wobei eine externe Oberfläche der proximalen Spitze (270) so geformt ist, dass eine erste und zweite Rille (276A, 276B) gebildet wird, die sich (a) axial entlang mindestens einem axialen Abschnitt der Spitze (270) erstrecken, und (b) so geformt und bemessen sind, um reversibel jeweilige proximale Endabschnitte (280A, 280B) des ersten und des zweiten sekundären Innenschafts (232A, 232B) aufzunehmen.

9. Vorrichtung gemäß einem der Ansprüche 1-2, wobei das Zustellwerkzeug (200) ferner mindestens ein Stenttransplantat-Stützglied (292) beinhaltet, das sicher an einer externen Oberfläche des primären Innenschafts (230) fixiert ist und das konfiguriert ist, um distale axiale Translation des Stenttransplantats (20) zu verhindern, wenn die externe Umhüllung (212) distal axial translatiert wird, um den Übergang des Stenttransplantats (20) vom radial komprimierten Zustand zum radial expandierten Zustand zu erleichtern.

10. Vorrichtung gemäß Anspruch 9, wobei das Stenttransplantat-Stützglied (292) umlaufend um den primären Innenschaft (230) angeordnet ist.

11. Vorrichtung gemäß Anspruch 9, wobei das Stenttransplantat-Stützglied (292) proximal neben dem proximalen Stenttransplantatende (28) positioniert ist, wenn das Stenttransplantat (20) innerhalb der externen Umhüllung (212) entfernbar positioniert ist, während es im radial komprimierten Zustand ist, mit dem proximalen Stenttransplantatende (28) den axialen Abschnitt (238) des primären Innenschafts (230) umgebend.

12. Vorrichtung gemäß einem der Ansprüche 1-2, wobei das Zustellwerkzeug (200) ferner eine proximale Spitze (270) beinhaltet, die mit einem proximalen Endabschnitt des primären Innenschafts (230) gekoppelt ist.

13. Vorrichtung gemäß Anspruch 12, wobei die proximale Spitze (270) konisch geformt ist, sodass die Spitze (270) eine kleinste Querschnittsfläche an einem proximalsten Abschnitt der Spitze (270) aufweist.

14. Vorrichtung gemäß Anspruch 12, wobei die proximale Spitze (270) halbkugelförmig geformt ist, sodass die Spitze (270) eine kleinste Querschnittsfläche an einem proximalsten Abschnitt der Spitze (270) aufweist.

## Revendications

1. Appareil comprenant un greffon-endoprothèse vasculaire globalement tubulaire (20), comportant des extrémités distale et proximale (26, 28) de greffon-endoprothèse vasculaire et comprenant :
un élément de support globalement tubulaire (30), qui comprend plusieurs éléments structuraux (31) d'endoprothèse vasculaire ; et
un élément de recouvrement (32) qui est assujetti à l'élément de support (30) et qui le recouvre au moins partiellement,
dans lequel, lorsque le greffon-endoprothèse vasculaire (20) est non contraint dans un état déployé radialement, une partie d'extrémité proximale (40) de l'élément de recouvrement (32) est formée de façon à définir au moins des première et deuxième pièces s'étendant de manière proximale (42A, 42B),
dans lequel, lorsque le greffon-endoprothèse vasculaire (20) est non contraint dans l'état déployé radialement et que les pièces s'étendant de manière proximale (42A, 42B) sont complètement déployées de manière proximale, les pièces s'étendant de manière proximale (42A, 42B) :
sont formées de façon à définir des bases distales respectives (46), qui (a) ont des longueurs respectives (L1) de base mesurées circonférentiellement autour du greffon-endoprothèse vasculaire (20), et (b) circonscrivent circonférentiellement des arcs de base respectifs (α), chacun desdits arcs de base (α) formant un angle compris entre 100 et 140 degrés,
sont formées de façon à définir des parties respectives les plus proximales (48), qui sont situées à une position plus proximale que toutes les autres parties respectives de l'élément de recouvrement (32) qui circonscrivent les arcs de base respectifs (a) de base, et
ont des longueurs axiales respectives (L2), mesurées axialement entre les bases distales respectives (46) et les parties les plus proximales respectives (48), lesdites longueurs (L2) ayant une valeur comprise entre 50 % et 150 % des longueurs de base respectives (L1), et
dans lequel, lorsque le greffon-endoprothèse vasculaire (20) est non contraint dans l'état déployé radialement et que les pièces s'étendant de manière proximale (42A, 42B) sont complètement déployées de manière proximale, les première et deuxième pièces s'étendant de manière proximale (42A, 42B) sont formées de façon à définir respectivement des première et seconde fenestrations (50), à travers l'élément de recouvrement (32), chacune desdites fenestrations (50) ayant une superficie d'au moins 6 mm²,
dans lequel l'appareil comprend en outre un outil de mise en place (200), qui (a) est conçu pour acheminer le greffon-endoprothèse vasculaire (20) dans l'état comprimé radialement jusqu'à une position cible dans la vasculature d'un sujet, et pour déployer le greffon-endoprothèse vasculaire (20) au niveau de la position cible, et (b) comprend :
une tige interne primaire (230), qui est formée de façon à définir un alésage primaire (220) ;
des première et seconde tiges internes secondaires (232A, 232B), qui sont formées de façon à définir respectivement des premier et second alésages secondaires (222A, 222B) les traversant ; et
une gaine externe (212), et
dans lequel l'outil de mise en place (200) est conçu de sorte qu'une translation axiale de la gaine externe (212) facilite une transition du greffon-endoprothèse vasculaire (20) de l'état comprimé radialement à un état déployé radialement,
**caractérisé en ce que** le greffon-endoprothèse vasculaire (20) est positionné de façon amovible, tout en se trouvant dans l'état comprimé radialement, à l'intérieur de la gaine externe (212), de sorte que :
(a) l'extrémité distale (26) de greffon-endoprothèse vasculaire entoure des parties axiales respectives (224) de la tige interne primaire (230) et des première et seconde tiges internes secondaires (232A, 232B),
(b) l'extrémité proximale (28) de greffon-endoprothèse vasculaire entoure une partie axiale (238) de la tige interne primaire (230), et
(c) les première et seconde tiges internes secondaires (232A, 232B) s'étendent de manière proximale au-delà de l'extrémité proximale (28) de greffon-endoprothèse vasculaire et traversent respectivement les première et seconde fenestrations (50).

2. Appareil selon la revendication 1, dans lequel les pièces s'étendant de manière proximale (42A, 42B) sont évasées en cône vers l'extérieur selon un angle compris entre 10 et 30 degrés par rapport à un axe longitudinal central du greffon-endoprothèse vasculaire (20), lorsque le greffon-endoprothèse vasculaire (20) est non contraint dans l'état déployé radialement et que les pièces s'étendant de manière proximale (42A, 42B) sont complètement déployées de manière proximale.

3. Appareil selon l'une quelconque des revendications 1 et 2, comprenant un système multicomposant (10) de greffon-endoprothèse vasculaire, comprenant :
le greffon-endoprothèse vasculaire (20) ; et
des première et seconde endoprothèses vasculaires de ramification recouvertes (22), comportant des parties d'extrémités respectives (52) qui sont dimensionnées et conçues pour former respectivement des joints d'étanchéité imperméables au sang avec les première et seconde fenestrations (50) des première et deuxième pièces s'étendant de manière proximale (42A, 42B) du greffon-endoprothèse vasculaire (20).

4. Appareil selon l'une ou l'autre des revendications 1 et 2, dans lequel chacune des première et seconde fenestrations (50) a une superficie inférieure ou égale à 35 mm² lorsque le greffon-endoprothèse vasculaire (20) est non contraint dans l'état déployé radialement et que les pièces s'étendant de manière proximale (42A, 42B) sont complètement déployées de manière proximale.

5. Appareil selon l'une ou l'autre des revendications 1 et 2, dans lequel les première et deuxième pièces s'étendant de manière proximale (42A, 42B) sont respectivement formées comme des premier et second lobes, lorsque le greffon-endoprothèse vasculaire (20) est non contraint dans l'état déployé radialement et que les pièces s'étendant de manière proximale (42A, 42B) sont complètement déployées de manière proximale.

6. Appareil selon l'une ou l'autre des revendications 1 et 2,
dans lequel, lorsque le greffon-endoprothèse vasculaire (20) est non contraint dans l'état déployé radialement, la partie d'extrémité proximale (40) de l'élément de recouvrement (32) est formée de façon à définir une troisième pièce s'étendant de manière proximale (42C), et
dans lequel, lorsque le greffon-endoprothèse vasculaire (20) est non contraint dans l'état déployé radialement et que la troisième pièce s'étendant de manière proximale (42C) est complètement déployée de manière proximale, la troisième pièce s'étendant de manière proximale (42C) :
est formée de façon à définir une troisième base distale (90), qui (a) a une troisième longueur de base mesurée circonférentiellement autour du greffon-endoprothèse vasculaire (20), et (b) circonscrit circonférentiellement un troisième arc de base, ledit troisième arc de base formant un angle compris entre 100 et 140 degrés,
est formée de façon à définir une troisième partie la plus proximale (92), qui se trouve à une position plus proximale que toutes les autres parties de l'élément de recouvrement (32) qui circonscrivent le troisième arc de base, et
a une troisième longueur axiale, mesurée axialement entre la troisième base distale (90) et la troisième partie la plus proximale (92), ladite longueur étant comprise entre 50 % et 150 % de la troisième longueur de base.

7. Appareil selon la revendication 6, dans lequel la troisième pièce s'étendant de manière proximale (42C) n'est pas formée de façon à définir de quelconques fenestrations à travers l'élément de recouvrement (32).

8. Appareil selon l'une ou l'autre des revendications 1 et 2,
dans lequel l'outil de mise en place (200) comprend en outre une pointe proximale (270) accouplée à une partie d'extrémité proximale de la tige interne primaire (230), et
dans lequel une surface externe de la pointe proximale (270) est formée de façon à définir des première et seconde rainures (276A, 276B), qui (a) s'étendent axialement le long d'au moins une partie axiale de la pointe (270), et (b) sont formées et dimensionnées de façon à recevoir de manière réversible des parties d'extrémités proximales respectives (280A, 280B) des première et seconde tiges internes secondaires (232A, 232B).

9. Appareil selon l'une ou l'autre des revendications 1 et 2, dans lequel l'outil de mise en place (200) comprend en outre au moins un élément de support (292) de greffon-endoprothèse vasculaire fixé de manière sûre à une surface externe de la tige interne primaire (230), et conçu pour empêcher une translation axiale distale du greffon-endoprothèse vasculaire (20) lorsque la gaine externe (212) fait l'objet d'une translation axiale distale pour faciliter la transition du greffon-endoprothèse vasculaire (20) de l'état comprimé radialement à l'état déployé radialement.

10. Appareil selon la revendication 9, dans lequel l'élément de support (292) de greffon-endoprothèse vasculaire est disposé circonférentiellement autour de la tige interne primaire (230).

11. Appareil selon la revendication 9, dans lequel l'élément de support (292) de greffon-endoprothèse vasculaire est positionné adjacent, de manière proximale, à l'extrémité proximale (28) de greffon-endoprothèse vasculaire, lorsque le greffon-endoprothèse vasculaire (20) est mis en place de manière amovible, tout en étant dans l'état comprimé radialement, à l'intérieur de la gaine externe (212), l'extrémité proximale (28) de greffon-endoprothèse vasculaire entourant la partie axiale (238) de la tige interne primaire (230).

12. Appareil selon l'une ou l'autre des revendications 1 et 2, dans lequel l'outil de mise en place (200) comprend en outre une pointe proximale (270) accouplée à une partie d'extrémité proximale de la tige interne primaire (230).

13. Appareil selon la revendication 12, dans lequel la pointe proximale (270) a une forme conique, de sorte que la pointe (270) ait une superficie de section transversale la plus petite au niveau d'une partie la plus proximale de la pointe (270).

14. Appareil selon la revendication 12, dans lequel la pointe proximale (270) a une forme hémisphérique, de sorte que la pointe (270) ait une superficie de section transversale la plus petite au niveau d'une partie la plus proximale de la pointe (270).
